# EUROPEAN PATENT SPECIFICATION

(11) **EP 3 601 363 B1**
(45) Date of publication and mention of the grant of the patent: **15.06.2022**
(21) Application number: 18776459.2
(22) Date of filing: 30.03.2018
(51) Int. Cl.: C07K 16/28, C07K 16/18, C07K 14/54, C07K 14/715, A61K 38/20, A61K 38/19, A61K 38/17, A61K 39/395, A61P 35/00, A61P 37/06

(54) **ALT-803 IN COMBINATION WITH ANTI-CD38 ANTIBODY FOR CANCER THERAPIES**
ALT-803 IN KOMBINATION MIT ANTI-CD38-ANTIKÖRPER FÜR KREBSTHERAPIEN
ALT-803 COMBINÉ À UN ANTICORPS ANTI-CD38 POUR THÉRAPIES ANTICANCÉREUSES

(30) Priority: 31.03.2017 US 201762480339 P; 27.03.2018 US 201815937493
(43) Date of publication of application: 05.02.2020
(73) Proprietor: Altor BioScience Corporation, Miramar, Florida 33025 (US)
(72) Inventor: SHRESTHA, Niraj, Miramar,FL 33025 (US); LIU, Bai, Cooper City, FL 33024 (US); RHODE, Peter, Miramar ,FL 33025 (US); WONG, Hing, C., Weston,FL 33332 (US)
(74) Representative: AWA Sweden AB
(86) International application number: PCT/US2018/025366
(87) International publication number: WO 2018/183821

(56) References cited:
- WO-A2-2016/004060
- US-A1- 2009 148 449
- US-A1- 2014 205 560
- PETER S. KIM ET AL: "IL-15 superagonist/IL-15R[alpha]Sushi-Fc fusion complex (IL-15SA/IL-15R[alpha]Su-Fc; ALT-803) markedly enhances specific subpopulations of NK and memory CD8+ T cells, and mediates potent anti-tumor activity against murine breast and colon carcinomas", ONCOTARGET, vol. 7, no. 13, 29 March 2016 (2016-03-29) , pages 16130-16145, XP055403855, United States ISSN: 1949-2553, DOI: 10.18632/oncotarget.7470
- MARIA CARMEN OCHOA ET AL: "Antibody-dependent cell cytotoxicity: immunotherapy strategies enhancing effector NK cells", IMMUNOLOGY AND CELL BIOLOGY, vol. 95, no. 4, 21 February 2017 (2017-02-21), pages 347-355, XP055510714, AU ISSN: 0818-9641, DOI: 10.1038/icb.2017.6
- FELICES, M et al.: "IL -15 super-agonist (ALT-803) enhances natural killer (NK) cell function against ovarian cancer", Gynecologic Oncology, vol. 145, no. 3, 22 February 2017 (2017-02-22), pages 453-461, XP085039603,
- LIU, B et al.: "A Novel Fusion of ALT-803 ( IL -15 Superagonist) with an Antibody Demonstrates Antigen-specific Antitumor Responses", Journal of Biological Chemistry, vol. 291, no. 46, 20 September 2016 (2016-09-20), pages 23869-23881, XP055501329,

## Description

### CROSS REFERENCE TO RELATED APPLICATIONS

This Application claims the benefit of U.S. Provisional Application 62/480,339 filed on March 31, 2017, and U.S. Application 15/937,493 filed on March 27, 2018.

### FIELD OF THE INVENTION

This invention relates generally to the field of therapies for treatment of cancer.

### BACKGROUND OF THE INVENTION

Prior to the invention described herein, there was a pressing need to develop new strategies to augment and/or direct immune activity against cancer.

US 2014/0205560 A1; Felices M et al., Gynecologic Oncology (2017) 145(3): 453-461; Liu B et al., Journal of Biologic Chemistry (2016) 291(46): 23869-23881; and Kim PS et al., Oncotarget (2016) 7(13): 16130-16145 disclose IL-15 agonists and IL-15/IL-15Ra complexes, such as ALT-803.

US 2009/0148449 A1 discloses human monoclonal antibodies which bind to human CD38 and therapeutic methods for using the human antibodies.

Ochoa MC et al., Immunology and Cell Biology (2017) 95(4):347-355 is a review article on therapeutic agents that stimulate antibody-dependent cell cytotoxicity (ADCC).

WO 2016/004060 A2 discloses the use of ALT-803, taken alone or in combination therapy, for treating neoplasia or an infection.

### SUMMARY OF THE INVENTION

The invention is based, at least in part, on the surprising discovery that an anti-CD38 antibody in combination with ALT-803, a complex of an interleukin-15 (IL-15) superagonist mutant and a dimeric IL-15 receptor α/Fc fusion protein, is useful for enhancing an immune response against a neoplasia (e.g., hematological cancer, B-cell neoplasm, multiple myeloma (MM), chronic lymphocytic leukemia (CLL), B and T acute lymphocytic leukemia, acute myeloid leukemia, chronic myeloid leukemia, hairy cell leukemia, Waldenstrom's macroglobulinemia, primary systemic amyloidosis, mantle cell lymphoma, NK cell leukemia, NK or T-cell lymphoma, plasma cell leukemia, B-cell lymphoma, non-Hodgkin's lymphoma, Burkitt's lymphoma, Hodgkin's lymphoma, and solid tumors).

A combination of (i) an anti-CD38 antibody or antibody-like molecule and (ii) an IL-15:IL-15Rα complex is provided for use in methods for treating a neoplasia in a subject, which are carried out by administering to the subject an effective amount of an anti-CD38 antibody (or antibody-like molecule) and an effective amount of the IL-15:IL-15Rα complex. A preferred IL-15:IL-15Rα complex is comprising an IL-15N72D:IL-15RαSu/Fc complex (ALT-803), wherein the ALT-803 comprises a dimeric IL-15RαSu/Fc and two IL-15N72D molecules. In one aspect, the IL-15N72D molecule comprises SEQ ID NO: 3. An exemplary IL-15RαSu/Fc comprises SEQ ID NO: 6.

The subject is preferably a mammal in need of such treatment, e.g., a subject that has been diagnosed with a neoplasia or a predisposition thereto. The mammal is any mammal, e.g., a human, a primate, a mouse, a rat, a dog, a cat, a horse, as well as livestock or animals grown for food consumption, e.g., cattle, sheep, pigs, chickens, and goats. In a preferred embodiment, the mammal is a human.

Suitable neoplasias for treatment with the methods described herein include hematological cancer, B-cell neoplasm, multiple myeloma (MM), chronic lymphocytic leukemia (CLL), B and T acute lymphocytic leukemia, acute myeloid leukemia, chronic myeloid leukemia, hairy cell leukemia, Waldenstrom's macroglobulinemia, primary systemic amyloidosis, mantle cell lymphoma, , NK cell leukemia, NK or T-cell lymphoma, plasma cell leukemia, B-cell lymphoma, non-Hodgkin's lymphoma, Burkitt's lymphoma, Hodgkin's lymphoma, and solid tumors.

Preferably, administration of the compositions described herein also prevents future recurrence of neoplasia after treatment of the disease. The therapeutic approaches described herein could also be combined with other agents that have been shown to be effective at treating MM including but not limited to dexamethasone, bortezomib, lenalidomide, thalidomide, bortezomib/doxil, carfilzomib, pomalidomide, panobinostat, elotuzumab, ixazomib, and glycosylated melphalan. The treatment approach of the invention could also be combined with any of the following therapies: radiation, chemotherapy, surgery, therapeutic antibodies, immunomodulatory agents, proteasome inhibitors, pan-DAC inhibitors, H-DAC inhibitors, checkpoint inhibitors, adoptive cell therapies include CAR T and NK cell therapy and vaccines. Additionally, these therapies could be used in treatment naive patients or those who have relapsed or refractory disease.

Additionally, pre- and post-medication strategies with steroids (i.e., corticosteroids, antipyretics and antihistamine) are typically be used in conjunction with ALT-803 plus anti-CD38 Ab therapy. Administration of anti-CD38 Ab often result in significant infusion reactions that may delay or terminate treatment and cause pain and discomfort to the patient. Steroids are used to reduce these effects but also may suppress immune responses necessary for therapeutic benefit. Surprisingly, ALT-803 plus anti-CD38 Ab therapy was found to be equally effective in animal tumor models in presence and absence of pre-treatment with steroids.

Exemplary effective doses of ALT-803 include between 0.1 µg/kg and 100 mg/kg body weight, e.g., 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, 45, 50, 55, 60, 65, 70, 75, 80, 85, 90, 95, 100, 200, 300, 400, 500, 600, 700, 800, or 900 µg/kg body weight or 1, 2, 3, 4, 5, 6, 7, 8, 9, 10, 20, 30, 40, 50, 60, 70, 80, 90, or 100 mg/kg body weight.

In some cases, the ALT-803 is administered daily, e.g., every 24 hours. Or, the ALT-803 is administered continuously or several times per day, e.g., every 1 hour, every 2 hours, every 3 hours, every 4 hours, every 5 hours, every 6 hours, every 7 hours, every 8 hours, every 9 hours, every 10 hours, every 11 hours, or every 12 hours.

Exemplary effective daily doses of ALT-803 include between 0.1 µg/kg and 100 µg/kg body weight, e.g., 0.1, 0.3, 0.5, 1, 5, 10, 15, 20, 25, 30, 35, 40, 45, 50, 55, 60, 65, 70, 75, 80, 85, 90, 95, or 99 µg/kg body weight.

Alternatively, the ALT-803 is administered about once per week, e.g., about once every 7 days. Or, the ALT-803 is administered twice per week, three times per week, four times per week, five times per week, six times per week, or seven times per week. Exemplary effective weekly doses of ALT-803 include between 0.0001 mg/kg and 4 mg/kg body weight, e.g., 0.001, 0.003, 0.005, 0.01. 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, or 4 mg/kg body weight. For example, an effective weekly dose of ALT-803 is between 0.1 µg/kg body weight and 400 µg/kg body weight. Alternatively, ALT-803 is administered at a fixed dose or based on body surface area (i.e., per m²).

In some cases, subjects receive two 6-week cycles consisting of 4 weekly ALT-803 intravenous doses followed by a 2-week rest period. Ultimately, the attending physician or veterinarian decides the appropriate amount and dosage regimen.

The compositions described herein are administered systemically, intravenously, subcutaneously, intramuscularly, intraperitoneally, intravesically, or by instillation. The antibody and ALT-803 may be administered simultaneously or sequentially.

The antibody (Ab) of the invention specifically binds to one or more epitopes on the CD38 protein (cyclic ADP ribose hydrolase). Preferred antibodies are composed of heavy and light chain immunoglobulin (Ig) proteins, which may include rodent, human, chimeric and humanized forms. Additionally, the method described herein could utilize antibody-like molecules, such as molecules comprising an antigen binding domain (e.g., single chain antibody, Fab, Fv, T-cell receptor binding domain, ligand binding domain or receptor binding domain). In some cases, these domains are preferably linked to an Fc domain. The Ig may be of any of the known isotypes (e.g., IgA, IgD, IgE, IgG, IgG1, IgG2, IgG3, IgG4, IgG2a, IgG2b, and IgM). In some applications described herein using anti-CD38 Abs (or antibody-like molecules), the Ab (or antibody-like molecule) contains a heavy chain or Fc domain capable of interacting with Fc receptors to mediate antibody-dependent cell-mediated cytotoxicity (ADCC) and/or antibody dependent cellular phagocytosis (ADCP). In other cases, antibodies conjugated to effector molecules may be used. In other applications, the preferred Ab (or antibody-like molecule) contains a heavy chain or Fc domain (e.g., IgG4 Fc) that is incapable of effectively mediating ADCC or ADCP. The anti-CD38 antibodies include but are not limited to daratumumab (DARZALEX^{®}), isatuximab (SAR650984), MOR202, Ab79, AB19, and MT-4019.

In one aspect, the addition of ALT-803 to anti-CD38 antibody treatment in vitro or in vivo increases cytotoxicity of immune cells against diseased or disease-associated cells. In some cases, ALT-803 is capable of stimulating immune cells to augment ADCC or ADCP activity against tumor cells mediated by a CD38-specific antibody (or antibody-like molecule). In one embodiment, treatment of immune cells with ALT-803 increases ADCC or ADCP activity against diseased of disease-associated cells mediated by an anti-CD38 antibody by at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. In a preferred embodiment, immune cells are treated with ALT-803 and used to kill tumor cells via ADCC or ADCP mediated by an anti-CD38 antibody, wherein the level of tumor cell death is at least 5% greater, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% greater than that seen with immune cells that were not treated with ALT-803. In preferred embodiments, tumor-specific ADCC or ADCP in the subject is augmented by at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% following the ALT-803 and antibody administration. In particular embodiments, NK cell-based ADCC activity is augmented by ALT-803 treatment.

In other cases, ALT-803 stimulates CD4⁺ and CD8⁺ T cells to kill diseased or disease-associated cells, e.g., tumor cells. Preferably, ALT-803 treatment stimulates immune cell cytolytic activity and anti-CD38 antibodies target disease cells, such that in combination these treatments provide highly effective and/or durable activity against the tumor cells. In some embodiments, ALT-803 increases serum levels of interferon gamma (IFN-γ) and/or IL-6, stimulates NK and T cell proliferation and upregulated NK and T cell expression of activation markers including CD25, CD69, perforin and granzyme. Induction of these markers may enhance the responsiveness or cytolytic activity of the immune cells against diseased cells. For example, the methods described herein stimulate NK cells to kill tumor cells.

In other embodiments, ALT-803 directly or indirectly induces the activity and/or level of other innate immune cells including neutrophils or monocytic cells. Such cells are known to mediate ADCC and ADCP of therapeutic antibodies against diseased cells, e.g., tumor cells (Golay, et al. Blood. 2013 122:3482-91, Richards, et al, Mol Cancer Ther 2008 7:2517-27). Preferably, combination therapy of ALT-803 and anti-CD38 antibodies provides improve clinical responses in patients with cancer through a mechanism that is mediated, at least in part, by innate immune cells. For example, the methods described herein stimulate neutrophils or monocytic cells to kill tumor cells. In one example, ALT-803 is capable of stimulating NK cells which produce cytokine or other agent that activate macrophage responses, including ADCP, thereby provide more effective tumor cell killing mediated by the anti-CD38 Ab.

Preferably, the methods described herein result in a reduced/decreased number of tumor cells compared to the number of tumor cells prior to administration of the compositions herein. Alternatively, the methods described herein result in a decreased disease progression of the neoplasia. Preferably, the methods described herein result in prolonged survival of a subject compared to untreated subjects.

Also provided is a kit for the treatment of a neoplasia, the kit comprising an effective amount of ALT-803, an anti-CD38 antibody, and directions for the use of the kit for the treatment of a neoplasia.

In certain aspects of the soluble fusion protein complexes of the invention, the IL-15 polypeptide is an IL-15 variant having a different amino acid sequence than native IL-15 polypeptide. The human IL-15 polypeptide is referred to herein as huIL-15, hIL-15, huIL15, hIL15, IL-15 wild type (wt), and variants thereof are referred to using the native amino acid, its position in the mature sequence and the variant amino acid. For example, huIL15N72D refers to human IL-15 comprising a substitution of N to D at position 72. In one aspect, the IL-15 variant functions as an IL-15 agonist as demonstrated, e.g., by increased binding activity for the IL-15RβγC receptors compared to the native IL-15 polypeptide. Alternatively, the IL-15 variant functions as an IL-15 antagonist as demonstrated by e.g., decreased binding activity for the IL-15RβγC receptors compared to the native IL-15 polypeptide.

Methods for killing a target cell are carried out by contacting a plurality of cells with an anti-CD38 antibody and ALT-803, wherein the plurality of cells further include immune cells bearing the IL-15R chains recognized by the IL-15 domain, or immune cells bearing Fc receptor chains recognized by the Fc domain, and the target cells bearing CD38 antigen recognized by an anti-CD38 antibody, and killing the target cells. For example, the target cells are tumor cells.

A method for killing diseased cells expressing CD38 antigen is carried out by treating immune cells with an effective amount of an IL-15N72D:IL-15RaSu/Fc complex (ALT-803), mixing the ALT-803-treated immune cells with an anti-CD38 antibody and diseased cells expressing CD38 antigen, and killing the diseased cells via ADCC or ADCP mediated by the ALT-803-treated immune cells and CD38-specific antibody. In one aspect of the method, the ALT-803-treated immune cells (i.e., NK cells) are capable of activating macrophage to mediate ADCP against tumor cells via anti-CD38 Ab. In one aspect, the level of diseased cell killing is increased by at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% compared to that mediated by immune cells that were not treated with ALT-803.

The combination of the invention is also useful in methods for preventing or treating a neoplasia in a patient in which the diseased cells express a CD38 antigen, the method including the steps of: contacting a plurality of cells with an anti-CD38 antibody and ALT-803, and damaging or killing the disease cells sufficient to prevent or treat the disease in the patient. In preferred embodiments, combination therapy with ALT-803 and an anti-CD38 antibody can decrease disease progression and/or prolong patient survival.

The combination of the invention is useful in methods of stimulating immune responses in a mammal by administering to the mammal an effective amount of an anti-CD38 antibody and an effective amount of ALT-803.

Specifically, methods for treating a neoplasia in a subject are carried out by administering to the subject an effective amount of an anti-CD38 antibody (or antibody-like molecule) and an effective amount of a pharmaceutical composition comprising an IL-15N72D:IL-15RaSu/Fc complex (ALT-803), wherein the ALT-803 comprises a dimeric IL-15RαSu/Fc and two IL-15N72D molecules, thereby treating the neoplasia. In some cases, the method further comprises treatment with a steroid before, during or after administration of said antibody or said ALT-803, wherein the steroid is selected from the group comprising corticosteroids, antipyretics and antihistamines. In one aspect, the IL-15N72D molecule comprises SEQ ID NO: 3. In another aspect, the IL-15RαSu/Fc comprises SEQ ID NO: 6. For example, the neoplasia is selected from the group consisting of a hematological cancer, B-cell neoplasm, multiple myeloma (MM), chronic lymphocytic leukemia (CLL), B and T acute lymphocytic leukemia, acute myeloid leukemia, chronic myeloid leukemia, hairy cell leukemia, Waldenstrom's macroglobulinemia, primary systemic amyloidosis, mantle cell lymphoma, NK cell leukemia, NK/T-cell lymphoma, plasma cell leukemia, B-cell lymphoma, non-Hodgkin's lymphoma, Burkitt's lymphoma, Hodgkin's lymphoma, and solid tumor.

Preferably, the effective amount of the ALT-803 is administered daily or once or twice per week. Optionally, the effective amount of said ALT-803 is between 0.1 µg/kg and 100 mg/kg. For example, the pharmaceutical composition is administered systemically, intravenously, subcutaneous, intramuscularly, intraperitoneally, intravesically, or by instillation. Exemplary anti-CD38 antibodies include daratumumab, isatuximab (SAR650984), and MOR202.

The antibody-dependent cell-mediated cytotoxicity (ADCC) or antibody dependent cellular phagocytosis (ADCP) mediated by the antibody against tumor cells in the subject is augmented by at least 5% following the administration, e.g., at least 5%, at least 10%, at least 15%, at least 20%, at least 25%, at least 30%, at least 35%, at least 40%, at least 50%, at least 60%, at least 70%, at least 80%, at least 90%, or at least 100%.

Preferably, the antibody and the ALT-803 increases levels of blood NK cell, T cell, neutrophil, macrophage, dendritic cell or monocyte counts or activity. In some cases, the antibody and the ALT-803 stimulate CD4⁺ or CD8⁺ T cells to kill tumor cells. In another case, the antibody and the ALT-803 stimulate NK cells to kill tumor cells. The antibody and the ALT-803 stimulate neutrophils or monocytic cells to kill tumor cells. The administration of the antibody and the ALT-803 results in a decrease in the number of tumor cells. Alternatively, or in addition, administration of the antibody and the ALT-803 results in a decrease in disease progression of the neoplasia. The administration of the antibody and the ALT-803 results in prolonged survival of the subjects compared to untreated subjects. Preferably, the subject is a human.

In one aspect, the antibody and the ALT-803 are administered simultaneously. In another aspect, the antibody and the ALT-803 are administered sequentially. Preferably, the administration prevents future recurrence of the neoplasia.

Also provided is a kit for the treatment of a neoplasia, the kit comprising an effective amount of ALT-803, an anti-CD38 antibody, and directions for the use of the kit for the treatment of a neoplasia.

Methods for killing diseased cells or disease-associated cells expressing CD38 antigen are carried out by treating immune cells with an effective amount of an IL-15N72D:IL-15RaSu/Fc complex (ALT-803), mixing the ALT-803-treated immune cells with an anti-CD38 antibody and diseased cells or disease-associated cells expressing CD38 antigen, and killing the diseased cells or disease-associated cells. For example, the level of diseased cell killing is increased by at least 5% compared to that mediated by immune cells that were not treated with ALT-803, e.g., 5%, 10%, 15%, 20%, 25%, 30%, 35%, 40%, 45%, 50%, 60%, 70%, 80%, 90%, or 100%. ALT-803-treated immune cells directly kill the diseased cells or disease-associated cells. In one aspect, ALT-803-treated immune cells stimulated other immune cells to kill the diseased cells or disease-associated cells. For example, killing of the diseased cells or disease-associated cells is mediated by ADCC or ADCP via the ALT-803-treated immune cells and the anti-CD38 antibody. Exemplary disease-associated cells include tumor cells.

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them below, unless specified otherwise.

By "agent" is meant a peptide, nucleic acid molecule, or small compound. An exemplary therapeutic agent is ALT-803.

By "ALT-803" is meant a complex comprising IL-15N72D noncovalently associated with a dimeric IL-15RaSu/Fc fusion protein and having immune stimulating activity. In one embodiment, the IL-15N72D and/or IL-15RαSu/Fc fusion protein comprises one, two, three, four or more amino acid variations relative to a reference sequence. An exemplary IL-15N72D amino acid sequence is provided below.

By "ameliorate" is meant decrease, suppress, attenuate, diminish, arrest, or stabilize the development or progression of a disease.

By "analog" is meant a molecule that is not identical, but has analogous functional or structural features. For example, a polypeptide analog retains the biological activity of a corresponding naturally-occurring polypeptide, while having certain biochemical modifications that enhance the analog's function relative to a naturally occurring polypeptide. Such biochemical modifications could increase the analog's protease resistance, membrane permeability, or half-life, without altering, for example, ligand binding. An analog may include an unnatural amino acid.

The invention includes antibodies or fragments of such antibodies, so long as they exhibit the desired biological activity. Also included in the invention are chimeric antibodies, such as humanized antibodies. Generally, a humanized antibody has one or more amino acid residues introduced into it from a source that is non-human. Humanization can be performed, for example, using methods described in the art, by substituting at least a portion of a rodent complementarity-determining region for the corresponding regions of a human antibody.

The term "antibody" (Ab) as used herein includes monoclonal antibodies, polyclonal antibodies, multispecific antibodies (e.g., bispecific antibodies), and antibody fragments, so long as they exhibit the desired biological activity. The term "immunoglobulin" (Ig) is used interchangeably with "antibody" herein.

By an "antibody-like" molecule is meant a molecule with a similar structure and/or a similar function as compared to that of an antibody. Exemplary antibody-like molecules include molecules comprising an antigen binding domain (e.g., single chain antibody, Fab, Fv, T-cell receptor binding domain, ligand binding domain or receptor binding domain).

An "isolated antibody" is one that has been separated and/or recovered from a component of its natural environment. Contaminant components of its natural environment are materials that would interfere with diagnostic or therapeutic uses for the antibody, and may include enzymes, hormones, and other proteinaceous or nonproteinaceous solutes. In preferred embodiments, the antibody is purified: (1) to greater than 95% by weight of antibody as determined by the Lowry method, and most preferably more than 99% by weight; (2) to a degree sufficient to obtain at least 15 residues of N-terminal or internal amino acid sequence by use of a spinning cup sequenator; or (3) to homogeneity by SDS-PAGE under reducing or non-reducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The basic four-chain antibody unit is a heterotetrameric glycoprotein composed of two identical light (L) chains and two identical heavy (H) chains. An IgM antibody consists of 5 of the basic heterotetramer unit along with an additional polypeptide called J chain, and therefore contains 10 antigen binding sites, while secreted IgA antibodies can polymerize to form polyvalent assemblages comprising 2-5 of the basic 4-chain units along with J chain. In the case of IgGs, the 4-chain unit is generally about 150,000 daltons. Each L chain is linked to an H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain (V_{H}) followed by three constant domains (C_{H}) for each of the α and γ chains and four C_{H} domains for µ and ε isotypes. Each L chain has at the N-terminus, a variable domain (V_{L}) followed by a constant domain (C_{L}) at its other end. The V_{L} is aligned with the V_{H} and the C_{L} is aligned with the first constant domain of the heavy chain (C_{H}1). Particular amino acid residues are believed to form an interface between the light chain and heavy chain variable domains. The pairing of a V_{H} and V_{L} together forms a single antigen-binding site. For the structure and properties of the different classes of antibodies, see, *e.g.,* Basic and Clinical Immunology, 8th edition, Daniel P. Stites, Abba I. Terr and Tristram G. Parslow (eds.), Appleton & Lange, Norwalk, Conn., 1994, page 71, and Chapter 6.

The L chain from any vertebrate species can be assigned to one of two clearly distinct types, called kappa (κ) and lambda (λ), based on the amino acid sequences of their constant domains (C_{L}). Depending on the amino acid sequence of the constant domain of their heavy chains (C_{H}), immunoglobulins can be assigned to different classes or isotypes. There are five classes of immunoglobulins: IgA, IgD, IgE, IgG, and IgM, having heavy chains designated alpha (α), delta (δ), epsilon (ε), gamma (γ) and mu (µ), respectively. The γ and α classes are further divided into subclasses on the basis of relatively minor differences in C_{H} sequence and function, e.g., humans express the following subclasses: IgG1, IgG2, IgG3, IgG4, IgA1, and IgA2.

The term "variable" refers to the fact that certain segments of the V domains differ extensively in sequence among antibodies. The V domain mediates antigen binding and defines specificity of a particular antibody for its particular antigen. However, the variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, Md. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody that are responsible for antigen binding. The hypervariable region generally comprises amino acid residues from a "complementarity determining region" or "CDR" (*e.g.,* around about residues 24-34 (L1), 50-56 (L2) and 89-97 (L3) in the V_{L}, and around about 31-35 (HI), 50-65 (H2) and 95-102 (H3) in the V_{H} when numbered in accordance with the Kabat numbering system; Kabat et al., Sequences of Proteins of Immunological Interest, 5th Ed. Public Health Service, National Institutes of Health, Bethesda, Md. (1991)); and/or those residues from a "hypervariable loop" (e.g., residues 24-34 (L1), 50-56 (L2) and 89-97 (L3) in the V_{L}, and 26-32 (HI), 52-56 (H2) and 95-101 (H3) in the V_{H} when numbered in accordance with the Chothia numbering system; Chothia and Lesk, J. Mol. Biol. 196:901-917 (1987)); and/or those residues from a "hypervariable loop"/CDR (e.g., residues 27-38 (L1), 56-65 (L2) and 105-120 (L3) in the V_{L}, and 27-38 (HI), 56-65 (H2) and 105-120 (H3) in the V_{H} when numbered in accordance with the IMGT numbering system; Lefranc, M.P. et al. Nucl. Acids Res. 27:209-212 (1999), Ruiz, M. e al. Nucl. Acids Res. 28:219-221 (2000)). Optionally, the antibody has symmetrical insertions at one or more of the following points 28, 36 (L1), 63, 74-75 (L2) and 123 (L3) in the V_{L}, and 28, 36 (HI), 63, 74-75 (H2) and 123 (H3) in the V_{H} when numbered in accordance with AHo; Honneger, A. and Plunkthun, A. J. Mol. Biol. 309:657-670 (2001)).

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to polyclonal antibody preparations that include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method. For example, the monoclonal antibodies useful in the present invention may be prepared by the hybridoma methodology first described by Kohler et al., Nature, 256:495 (1975), or may be made using recombinant DNA methods in bacterial, eukaryotic animal or plant cells (see, *e.g.,* U.S. Pat. No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example.

Monoclonal antibodies include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see U.S. Pat. No. 4,816,567; and Morrison et al., Proc. Natl. Acad. Sci. USA, 81:6851-6855 (1984)). Also provided are variable domain antigen-binding sequences derived from human antibodies. Accordingly, chimeric antibodies of primary interest herein include antibodies having one or more human antigen binding sequences (e.g., CDRs) and containing one or more sequences derived from a non-human antibody, e.g., an FR or C region sequence. In addition, chimeric antibodies of primary interest herein include those comprising a human variable domain antigen binding sequence of one antibody class or subclass and another sequence, *e.g.,* FR or C region sequence, derived from another antibody class or subclass. Chimeric antibodies of interest herein also include those containing variable domain antigen-binding sequences related to those described herein or derived from a different species, such as a non-human primate *(e.g.,* Old World Monkey, Ape, etc.). Chimeric antibodies also include primatized and humanized antibodies.

Furthermore, chimeric antibodies may comprise residues that are not found in the recipient antibody or in the donor antibody. These modifications are made to further refine antibody performance. For further details, see Jones et al., Nature 321:522-525 (1986); Riechmann et al., Nature 332:323-329 (1988); and Presta, Curr. Op. Struct. Biol. 2:593-596 (1992).

A "humanized antibody" is generally considered to be a human antibody that has one or more amino acid residues introduced into it from a source that is non-human. These non-human amino acid residues are often referred to as "import" residues, which are typically taken from an "import" variable domain. Humanization is traditionally performed following the method of Winter and co-workers (Jones et al., Nature, 321:522-525 (1986); Reichmann et al., Nature, 332:323-327 (1988); Verhoeyen et al., Science, 239:1534-1536 (1988)), by substituting import hypervariable region sequences for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species.

A "human antibody" is an antibody containing only sequences present in an antibody naturally produced by a human. However, as used herein, human antibodies may comprise residues or modifications not found in a naturally occurring human antibody, including those modifications and variant sequences described herein. These are typically made to further refine or enhance antibody performance.

An "intact" antibody is one that comprises an antigen-binding site as well as a C_{L} and at least heavy chain constant domains, C_{H} 1, C_{H} 2 and C_{H} 3. The constant domains may be native sequence constant domains (e.g., human native sequence constant domains) or amino acid sequence variant thereof. Preferably, the intact antibody has one or more effector functions.

An "antibody fragment" comprises a portion of an intact antibody, preferably the antigen binding or variable region of the intact antibody. Examples of antibody fragments include Fab, Fab', F(ab')₂, and Fv fragments; diabodies; linear antibodies (see U.S. Pat. No. 5,641,870; Zapata et al., Protein Eng. 8(10): 1057-1062 [1995]); single-chain antibody molecules; and multispecific antibodies formed from antibody fragments.

The phrase "functional fragment or analog" of an antibody is a compound having qualitative biological activity in common with a full-length antibody. For example, a functional fragment or analog of an anti-IgE antibody is one that can bind to an IgE immunoglobulin in such a manner so as to prevent or substantially reduce the ability of such molecule from having the ability to bind to the high affinity receptor, Fc_{ε}RI.

Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (V_{H}), and the first constant domain of one heavy chain (C_{H} 1). Each Fab fragment is monovalent with respect to antigen binding, *i.e.,* it has a single antigen-binding site. Pepsin treatment of an antibody yields a single large F(ab')₂ fragment that roughly corresponds to two disulfide linked Fab fragments having divalent antigen-binding activity and is still capable of cross-linking antigen. Fab' fragments differ from Fab fragments by having additional few residues at the carboxy terminus of the C_{H}1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments that have hinge cysteines between them. Other chemical couplings of antibody fragments are also known.

The "Fc" fragment comprises the carboxy-terminal portions of both H chains held together by disulfides. The effector functions of antibodies are determined by sequences in the Fc region, which region is also the part recognized by Fc receptors (FcR) found on certain types of cells.

"Fv" is the minimum antibody fragment that contains a complete antigen-recognition and -binding site. This fragment consists of a dimer of one heavy- and one light-chain variable region domain in tight, non-covalent association. From the folding of these two domains emanate six hypervariable loops (three loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv" also abbreviated as "sFv" or "scFv" are antibody fragments that comprise the V_{H} and V_{L} antibody domains connected into a single polypeptide chain. Preferably, the sFv polypeptide further comprises a polypeptide linker between the V_{H} and V_{L} domains that enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, *infra.*

The term "diabodies" refers to small antibody fragments prepared by constructing sFv fragments (see preceding paragraph) with short linkers (about 5-10 residues) between the V_{H} and V_{L} domains such that inter-chain but not intra-chain pairing of the V domains is achieved, resulting in a bivalent fragment, *i.e.,* fragment having two antigen-binding sites. Bispecific diabodies are heterodimers of two "crossover" sFv fragments in which the V_{H} and V_{L} domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

As used herein, an antibody that "internalizes" is one that is taken up by *(i.e.,* enters) the cell upon binding to an antigen on a mammalian cell *(e.g.,* a cell surface polypeptide or receptor). The internalizing antibody will of course include antibody fragments, human or chimeric antibody, and antibody conjugates. For certain therapeutic applications, internalization *in vivo* is contemplated. The number of antibody molecules internalized will be sufficient or adequate to kill a cell or inhibit its growth, especially an infected cell. Depending on the potency of the antibody or antibody conjugate, in some instances, the uptake of a single antibody molecule into the cell is sufficient to kill the target cell to which the antibody binds. For example, certain toxins are highly potent in killing such that internalization of one molecule of the toxin conjugated to the antibody is sufficient to kill the infected cell.

As used herein, an antibody is said to be "immunospecific," "specific for" or to "specifically bind" an antigen if it reacts at a detectable level with the antigen, preferably with an affinity constant, K_{a,} of greater than or equal to about 10⁴ M⁻¹, or greater than or equal to about 10⁵ M⁻¹, greater than or equal to about 10⁶ M⁻¹, greater than or equal to about 10⁷ M⁻¹, or greater than or equal to 10⁸ M⁻¹. Affinity of an antibody for its cognate antigen is also commonly expressed as a dissociation constant K_{D}, and in certain embodiments, HuM2e antibody specifically binds to M2e if it binds with a K_{D} of less than or equal to 10⁻⁴ M, less than or equal to about 10⁻⁵ M, less than or equal to about 10⁻⁶ M, less than or equal to 10⁻⁷ M, or less than or equal to 10⁻⁸ M. Affinities of antibodies can be readily determined using conventional techniques, for example, those described by Scatchard et al. (Ann. N. Y. Acad. Sci. USA 51:660 (1949)).

Binding properties of an antibody to antigens, cells or tissues thereof may generally be determined and assessed using immunodetection methods including, for example, immunofluorescence-based assays, such as immuno-histochemistry (IHC) and/or fluorescence-activated cell sorting (FACS).

An antibody having a "biological characteristic" of a designated antibody is one that possesses one or more of the biological characteristics of that antibody which distinguish it from other antibodies. For example, in certain embodiments, an antibody with a biological characteristic of a designated antibody will bind the same epitope as that bound by the designated antibody and/or have a common effector function as the designated antibody. The term "antagonist" antibody is used in the broadest sense, and includes an antibody that partially or fully blocks, inhibits, or neutralizes a biological activity of an epitope, polypeptide, or cell that it specifically binds. Methods for identifying antagonist antibodies may comprise contacting a polypeptide or cell specifically bound by a candidate antagonist antibody with the candidate antagonist antibody and measuring a detectable change in one or more biological activities normally associated with the polypeptide or cell.

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors *(e.g.,* B cell receptor); and B cell activation.

By "binding to" a molecule is meant having a physicochemical affinity for that molecule.

By "control" or "reference" is meant a standard of comparison. In one aspect, as used herein, "changed as compared to a control" sample or subject is understood as having a level that is statistically different than a sample from a normal, untreated, or control sample. Control samples include, for example, cells in culture, one or more laboratory test animals, or one or more human subjects. Methods to select and test control samples are within the ability of those in the art. An analyte can be a naturally occurring substance that is characteristically expressed or produced by the cell or organism (e.g., an antibody, a protein) or a substance produced by a reporter construct (e.g, β-galactosidase or luciferase). Depending on the method used for detection, the amount and measurement of the change can vary. Determination of statistical significance is within the ability of those skilled in the art, e.g., the number of standard deviations from the mean that constitute a positive result.

"Detect" refers to identifying the presence, absence or amount of the analyte to be detected.

By "disease" is meant any condition or disorder that damages or interferes with the normal function of a cell, tissue, or organ. Examples of diseases include neoplasias.

By the terms "effective amount" and "therapeutically effective amount" of a formulation or formulation component is meant a sufficient amount of the formulation or component, alone or in a combination, to provide the desired effect. For example, by "an effective amount" is meant an amount of a compound, alone or in a combination, required to ameliorate the symptoms of a disease relative to an untreated patient. The effective amount of active compound(s) used to practice the present invention for therapeutic treatment of a disease varies depending upon the manner of administration, the age, body weight, and general health of the subject. Ultimately, the attending physician or veterinarian will decide the appropriate amount and dosage regimen. Such amount is referred to as an "effective" amount.

By "fragment" is meant a portion of a polypeptide or nucleic acid molecule. This portion contains, preferably, at least 10%, 20%, 30%, 40%, 50%, 60%, 70%, 80%, or 90% of the entire length of the reference nucleic acid molecule or polypeptide. For example, a fragment may contain 10, 20, 30, 40, 50, 60, 70, 80, 90, or 100, 200, 300, 400, 500, 600, 700, 800, 900, or 1000 nucleotides or amino acids. However, the invention also comprises polypeptides and nucleic acid fragments, so long as they exhibit the desired biological activity of the full length polypeptides and nucleic acid, respectively. A nucleic acid fragment of almost any length is employed. For example, illustrative polynucleotide segments with total lengths of about 10,000, about 5000, about 3000, about 2,000, about 1,000, about 500, about 200, about 100, about 50 base pairs in length (including all intermediate lengths) are included in many implementations of this invention. Similarly, a polypeptide fragment of almost any length is employed. For example, illustrative polypeptide segments with total lengths of about 10,000, about 5,000, about 3,000, about 2,000, about 1,000, about 5,000, about 1,000, about 500, about 200, about 100, or about 50 amino acids in length (including all intermediate lengths) are included in many implementations of this invention.

The terms "isolated", "purified", or "biologically pure" refer to material that is free to varying degrees from components which normally accompany it as found in its native state. "Isolate" denotes a degree of separation from original source or surroundings. "Purify" denotes a degree of separation that is higher than isolation.

A "purified" or "biologically pure" protein is sufficiently free of other materials such that any impurities do not materially affect the biological properties of the protein or cause other adverse consequences. That is, a nucleic acid or peptide as disclosed herein is purified if it is substantially free of cellular material, viral material, or culture medium when produced by recombinant DNA techniques, or chemical precursors or other chemicals when chemically synthesized. Purity and homogeneity are typically determined using analytical chemistry techniques, for example, polyacrylamide gel electrophoresis or high performance liquid chromatography. The term "purified" can denote that a nucleic acid or protein gives rise to essentially one band in an electrophoretic gel. For a protein that can be subjected to modifications, for example, phosphorylation or glycosylation, different modifications may give rise to different isolated proteins, which can be separately purified.

Similarly, by "substantially pure" is meant a nucleotide or polypeptide that has been separated from the components that naturally accompany it. Typically, the nucleotides and polypeptides are substantially pure when they are at least 60%, 70%, 80%, 90%, 95%, or even 99%, by weight, free from the proteins and naturally-occurring organic molecules with they are naturally associated.

By "isolated nucleic acid" is meant a nucleic acid that is free of the genes which flank it in the naturally-occurring genome of the organism from which the nucleic acid is derived. The term covers, for example: (a) a DNA which is part of a naturally occurring genomic DNA molecule, but is not flanked by both of the nucleic acid sequences that flank that part of the molecule in the genome of the organism in which it naturally occurs; (b) a nucleic acid incorporated into a vector or into the genomic DNA of a prokaryote or eukaryote in a manner, such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein. Isolated nucleic acid molecules disclosed herein further include molecules produced synthetically, as well as any nucleic acids that have been altered chemically and/or that have modified backbones. For example, the isolated nucleic acid is a purified cDNA or RNA polynucleotide. Isolated nucleic acid molecules also include messenger ribonucleic acid (mRNA) molecules.

By an "isolated polypeptide" is meant a polypeptide of the invention that has been separated from components that naturally accompany it. Typically, the polypeptide is isolated when it is at least 60%, by weight, free from the proteins and naturally-occurring organic molecules with which it is naturally associated. Preferably, the preparation is at least 75%, more preferably at least 90%, and most preferably at least 99%, by weight, a polypeptide of the invention. An isolated polypeptide of the invention may be obtained, for example, by extraction from a natural source, by expression of a recombinant nucleic acid encoding such a polypeptide; or by chemically synthesizing the protein. Purity can be measured by any appropriate method, for example, column chromatography, polyacrylamide gel electrophoresis, or by HPLC analysis.

By "marker" is meant any protein or polynucleotide having an alteration in expression level or activity that is associated with a disease or disorder.

By "neoplasia" is meant a disease or disorder characterized by excess proliferation or reduced apoptosis. Illustrative neoplasms for which the invention can be used include, but are not limited to leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (Hodgkin's disease, non-Hodgkin's disease), Waldenstrom's macroglobulinemia, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, gastric and esophageal cancer, head and neck cancer, rectal cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, glioblastoma multiforme, astrocytoma, medulloblastoma, craniopharyngioma, ependymoma, pinealoma, hemangioblastoma, acoustic neuroma, oligodenroglioma, schwannoma, meningioma, melanoma, neuroblastoma, and retinoblastoma). In particular embodiments, the neoplasia is multiple myeloma, beta-cell lymphoma, urothelial/bladder carcinoma or melanoma. As used herein, "obtaining" as in "obtaining an agent" includes synthesizing, purchasing, or otherwise acquiring the agent.

By "reduces" is meant a negative alteration of at least 5%, 10%, 25%, 50%, 75%, or 100%.

A "reference sequence" is a defined sequence used as a basis for sequence comparison. A reference sequence may be a subset of or the entirety of a specified sequence; for example, a segment of a full-length cDNA or gene sequence, or the complete cDNA or gene sequence. For polypeptides, the length of the reference polypeptide sequence will generally be at least about 16 amino acids, preferably at least about 20 amino acids, more preferably at least about 25 amino acids, and even more preferably about 35 amino acids, about 50 amino acids, or about 100 amino acids. For nucleic acids, the length of the reference nucleic acid sequence will generally be at least about 50 nucleotides, preferably at least about 60 nucleotides, more preferably at least about 75 nucleotides, and even more preferably about 100 nucleotides or about 300 nucleotides or any integer thereabout or therebetween.

By "specifically binds" is meant a compound or antibody that recognizes and binds a polypeptide of the invention, but which does not substantially recognize and bind other molecules in a sample, for example, a biological sample, which naturally includes a polypeptide of the invention.

Nucleic acid molecules useful in the present disclosure include any nucleic acid molecule that encodes a polypeptide as disclosed herein or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule. Nucleic acid molecules useful in the present disclosure include any nucleic acid molecule that encodes a polypeptide as disclosed herein or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule. By "hybridize" is meant pair to form a double-stranded molecule between complementary polynucleotide sequences (e.g., a gene described herein), or portions thereof, under various conditions of stringency. (See, e.g., Wahl, G. M. and S. L. Berger (1987) Methods Enzymol. 152:399; Kimmel, A. R. (1987) Methods Enzymol. 152:507).

For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30° C, more preferably of at least about 37° C, and most preferably of at least about 42° C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred: embodiment, hybridization will occur at 30° C in 750 mM NaCl, 75 mM trisodium citrate, and 1% SDS. In a more preferred embodiment, hybridization will occur at 37° C in 500 mM NaCl, 50 mM trisodium citrate, 1% SDS, 35% formamide, and 100 .mu.g/ml denatured salmon sperm DNA (ssDNA). In a most preferred embodiment, hybridization will occur at 42° C in 250 mM NaCl, 25 mM trisodium citrate, 1% SDS, 50% formamide, and 200 µg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25° C, more preferably of at least about 42° C, and even more preferably of at least about 68° C. In a preferred embodiment, wash steps will occur at 25° C in 30 mM NaCl, 3 mM trisodium citrate, and 0.1% SDS. In a more preferred embodiment, wash steps will occur at 42 C in 15 mM NaCl, 1.5 mM trisodium citrate, and 0.1% SDS. In a more preferred embodiment, wash steps will occur at 68° C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

By "substantially identical" is meant a polypeptide or nucleic acid molecule exhibiting at least 50% identity to a reference amino acid sequence (for example, any one of the amino acid sequences described herein) or nucleic acid sequence (for example, any one of the nucleic acid sequences described herein). Preferably, such a sequence is at least 60%, more preferably 80% or 85%, and more preferably 90%, 95% or even 99% identical at the amino acid level or nucleic acid to the sequence used for comparison.

Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e⁻³ and e⁻¹⁰⁰ indicating a closely related sequence.

By "subject" is meant a mammal, including, but not limited to, a human or non-human mammal, such as a bovine, equine, canine, ovine, or feline. The subject is preferably a mammal in need of such treatment, e.g., a subject that has been diagnosed with B cell lymphoma or a predisposition thereto. The mammal is any mammal, e.g., a human, a primate, a mouse, a rat, a dog, a cat, a horse, as well as livestock or animals grown for food consumption, e.g., cattle, sheep, pigs, chickens, and goats. In a preferred embodiment, the mammal is a human.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

The terms "treating" and "treatment" as used herein refer to the administration of an agent or formulation to a clinically symptomatic individual afflicted with an adverse condition, disorder, or disease, so as to effect a reduction in severity and/or frequency of symptoms, eliminate the symptoms and/or their underlying cause, and/or facilitate improvement or remediation of damage. It will be appreciated that, although not precluded, treating a disorder or condition does not require that the disorder, condition or symptoms associated therewith be completely eliminated.

Treatment of patients with neoplasia may include any of the following: Adjuvant therapy (also called adjunct therapy or adjunctive therapy) to destroy residual tumor cells that may be present after the known tumor is removed by the initial therapy (e.g. surgery), thereby preventing possible cancer reoccurrence; neoadjuvant therapy given prior to the surgical procedure to shrink the cancer; induction therapy to cause a remission, typically for acute leukemia; consolidation therapy (also called intensification therapy) given once a remission is achieved to sustain the remission; maintenance therapy given in lower or less frequent doses to assist in prolonging a remission; first line therapy (also called standard therapy); second (or 3rd, 4th, etc.) line therapy (also called salvage therapy) is given if a disease has not responded or reoccurred after first line therapy; and palliative therapy (also called supportive therapy) to address symptom management without expecting to significantly reduce the cancer.

The terms "preventing" and "prevention" refer to the administration of an agent or composition to a clinically asymptomatic individual who is susceptible or predisposed to a particular adverse condition, disorder, or disease, and thus relates to the prevention of the occurrence of symptoms and/or their underlying cause.

Unless specifically stated or obvious from context, as used herein, the term "or" is understood to be inclusive. Unless specifically stated or obvious from context, as used herein, the terms "a", "an", and "the" are understood to be singular or plural.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

Other features and advantages of the invention will be apparent from the following description of the preferred embodiments thereof, and from the claims. Unless otherwise defined, all technical and scientific terms used herein have the same meaning as commonly understood by one of ordinary skill in the art to which this invention belongs. Although methods and materials similar to those described herein can be used in the practice of the present invention, suitable methods and materials are described below. The materials, methods, and examples are illustrative only and not intended to be limiting.

### BRIEF DESCRIPTION OF THE DRAWINGS

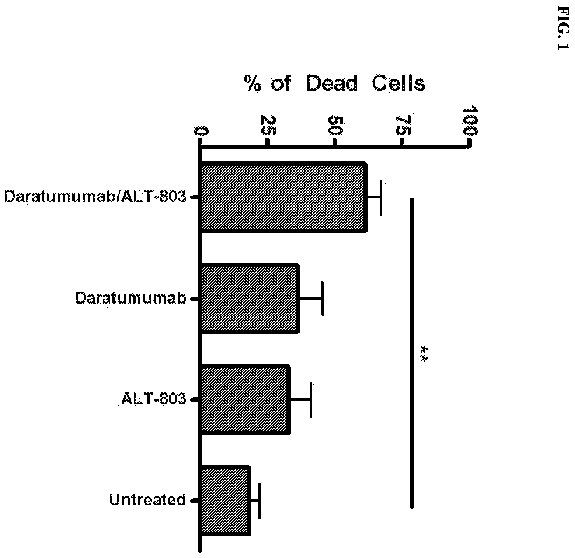

FIG. 1 is a bar graph demonstrating the effects of ALT-803 on human peripheral blood mononuclear cell (PBMC) antibody-dependent cell-mediated cytotoxicity (ADCC) directed anti-CD38 Ab against hematologic tumor cells. Human PBMC were used as effector cells and CellTrace Violet labeled Daudi cells were targets (E:T ratio 5:1) with no stimulation (Untreated), ALT-803 (10 nM), anti-CD38 Ab (10 nM) or ALT-803 (10 nM) plus anti-CD38 Ab (10 nM). After 2 days, Daudi cell death (positive staining with propidium iodide) was determined by flow cytometry. The bars represent the mean ± SEM for PMBCs from 4 independent donors.

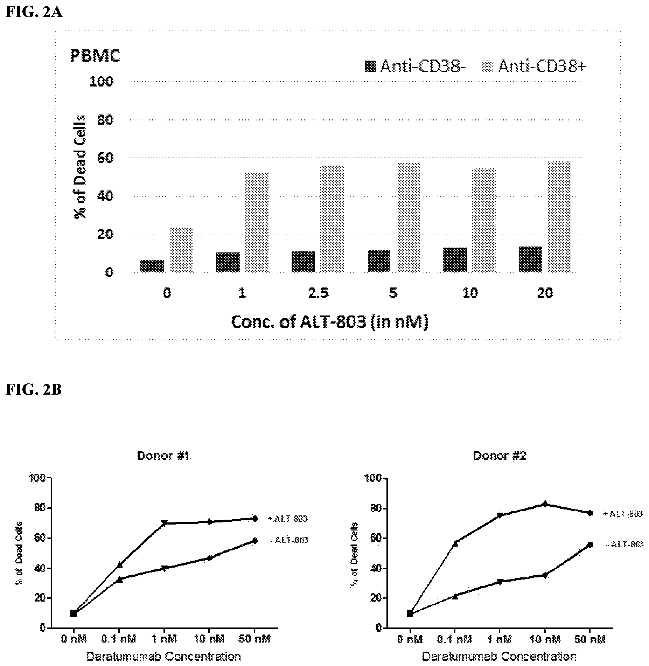

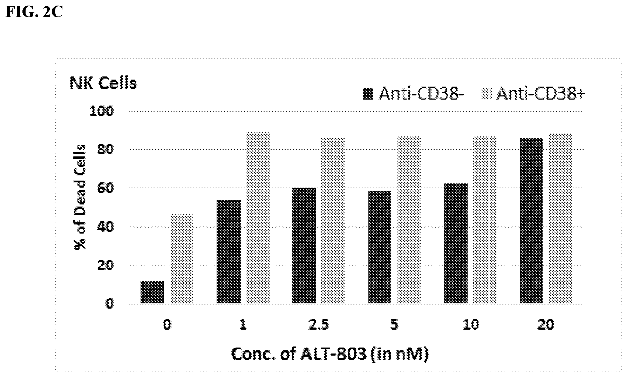

FIG. 2A is a bar graph demonstrating the concentration dependent effects of ALT-803 on human PBMC ADCC directed anti-CD38 Ab against hematologic tumor cells. Freshly isolated PBMCs were incubated with different concentrations of ALT-803 with and without 10 nM anti-CD38 Ab. After 2 days, ADCC activity against Daudi tumor target cells (E:T ratio 2:1) was determined as described in FIG. 1. The bars represent the mean ± SEM for PMBCs from 4 independent donors. FIG. 2B shows line graphs demonstrating the concentration dependent effects of anti-CD38 Ab with or without 10 nM ALT-803 on human PBMC ADCC directed against Daudi cells using the method described in FIG 2A. Results from 2 different donors are shown, FIG. 2C is a bar graph demonstrating the concentration dependent effects of ALT-803 on human natural killer (NK) cell ADCC directed anti-CD38 Ab against hematologic tumor cells. Freshly purified human NK cells (>90% CD56⁺CD3⁻) were incubated with different concentrations of ALT-803 with and without 10 nM anti-CD38 Ab. After 2 days, ADCC activity against Daudi tumor target cells (E:T ratio 2:1) was determined as described in FIG. 1. The bars represent the mean ± SEM for NK cells from 4 independent donors.

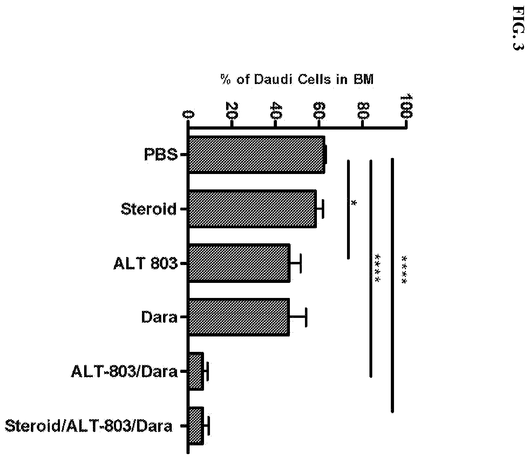

FIG. 3 is a bar graph showing the efficacy of ALT-803 plus anti-CD38 Ab treatment in reducing tumor burden in the bone marrow of mice bearing Daudi cell tumors. Severe combined immunodeficiency (SCID) mice were injected (i.v.) on day 0 with 1 × 10⁷ Daudi cells. Tumor-bearing mice (6 mice in each group) were treated i.v. with PBS (vehicle, i.v.), anti-CD38 Ab (10 mg/kg, i.v.), ALT-803 (0.2 mg/kg, subcutaneously), or anti-CD38 Ab (10 mg/kg) plus ALT-803 (0.2 mg/kg, subcutaneously). Anti-CD38 Ab (Dara) was administered on day 5 post tumor cell inoculation and ALT-803 was administered on day 17 post tumor cell inoculation. Some mice were also treated with steroid (0.2 µg hydrocortisone /dose i.p.) on day 15 as indicated. Percentage of Daudi cells (human HLA-DR⁺) in the bone marrow (BM) at day 22 post-injection was quantified by flow cytometer. ^{∗}, P < 0.05; ^{∗∗∗∗} P <0.0001.

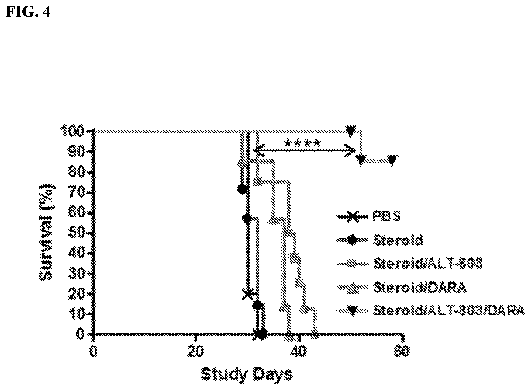

FIG. 4 is a line graph demonstrating that the combination of ALT-803 and anti-CD38 Ab is capable of prolonging survival of mice bearing Daudi cell tumors. SCID mice were injected (i.v.) on day 0 with 1 × 10⁷ Daudi cells. Tumor-bearing mice (6 mice in each group) were treated i.v. with phosphate-buffered saline (PBS) (vehicle, i.v.), anti-CD38 Ab (10 mg/kg, i.v.), ALT-803 (0.2 mg/kg, subcutaneously), or anti-CD38 Ab (10 mg/kg) plus ALT-803 (0.2 mg/kg, subcutaneously). All groups of mice received steroid (0.2 mg/mouse, intraperitoneal) to reduce pre-infusion reaction except PBS group mice. Anti-CD38 Ab (DARA) was administered on day 5 post tumor cell inoculation and ALT-803 was administered on day 17 post tumor cell inoculation. On day 22, the groups received a second treatment of PBS (vehicle, i.v.), anti-CD38 Ab (10 mg/kg, i.v.), ALT-803 (0.2 mg/kg, subcutaneously), or anti-CD38 Ab (10 mg/kg) plus ALT-803 (0.2 mg/kg, subcutaneously). Mice were not treated with steroid on day 22. Mouse survival was monitored and Kaplan-Meier analysis is shown. ^{∗∗∗∗} P <0.0001.

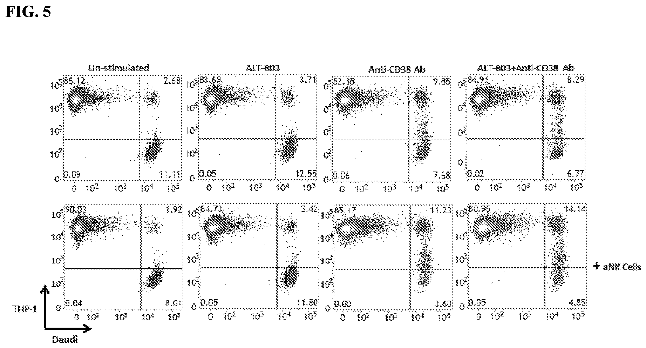

FIG. 5 shows a series of flow cytometry plots demonstrating the effects of ALT-803 in combination with anti-CD38 Ab on macrophage-mediated phagocytosis of Daudi tumor cells in vivo. PKH67-labeled human THP-1 macrophage cells and CellTrace Violet-labeled Daudi cells were co-cultured (E:T ratio of 1:1) with PBS (US), 10 nM anti-CD38 Ab and/or 10 nM ALT-803 in presence or absence of NK-92 cells (aNK cells). After 24 hours, the percentage of phagocytosed Daudi tumor cells (represented by PKH67⁺ CellTrace Violet⁺ THP-1 cells in upper right quadrant) was measured by flow cytometry.

### DETAILED DESCRIPTION

The invention is based, at least in part, on the surprising discovery that an anti-CD38 antibody in combination with ALT-803, a complex of an interleukin-15 (IL-15) superagonist mutant and a dimeric IL-15 receptor α/Fc fusion protein, is useful for enhancing an immune response against a neoplasia (e.g., a glioblastoma, prostate cancer, hematological cancer, B-cell neoplasms, multiple myeloma, B-cell lymphoma, Hodgkin's lymphoma, acute myeloid leukemia, chronic lymphocytic leukemia, cutaneous T-cell lymphoma, T-cell lymphoma, a solid tumor, urothelial/bladder carcinoma, melanoma, lung cancer, renal cell carcinoma, breast cancer, head and neck cancer, colorectal cancer, and ovarian cancer).

### ALT-803

ALT-803 comprises an IL-15 mutant with increased ability to bind IL-2Rβγ and enhanced biological activity (U.S. Patent No. 8,507, 222). This super agonist mutant of IL-15 was described in a publication (J Immunol 2009 183:3598) and a patent has been issued by the U.S. Patent & Trademark Office on the super agonist and several patents applications are pending (e.g., U.S. Ser. No. 12/151,980 and 13/238,925). This IL-15 super agonist in combination with a soluble IL-15α receptor fusion protein (IL-15RaSu/Fc) results in a protein complex with highly potent IL-15 activity in vitro and in vivo (Han et al., 2011, Cytokine, 56: 804-810; Xu, et al., 2013 Cancer Res. 73:3075-86, Wong, et al., 2013, OncoImmunology 2:e26442). This IL-15 super agonist complex (IL-15N72D:IL-15RaSu/Fc) is referred to as ALT-803. Pharmacokinetic analysis indicated that the complex has a half-life in mice of 25 hours following i.v. administration. ALT-803 exhibits impressive anti-tumor activity against aggressive solid and hematological tumor models in immunocompetent mice. It can be administered as a monotherapy using a twice weekly or weekly i.v. dose regimen or as combinatorial therapy with an antibody. The ALT-803 anti-tumor response is also durable. Tumor-bearing mice that were cured after ALT-803 treatment were also highly resistant to re-challenge with the same tumor cells indicating that ALT-803 induces effective immunological memory responses against the re-introduced tumor cells.

### Interleukin-15

Interleukin-15 (IL-15) is an important cytokine for the development, proliferation, and activation of effector NK cells and CD8⁺ memory T cells. IL-15 binds to the IL-15 receptor α (IL-15Rα) and is presented in *trans* to the IL-2/IL-15 receptor β - common γ chain (IL-15Rβγ_{c}) complex on effector cells. IL-15 and IL-2 share binding to the IL-15Rβγ_{c}, and signal through STAT3 and STAT5 pathways. However, IL-2 also supports maintenance of CD4⁺CD25⁺FoxP3⁺ regulatory T (Treg) cells and induces cell death of activated CD8⁺ T cells. These effects may limit the therapeutic activity of IL-2 against tumors. IL-15 does not share these immunosuppressive activities with IL-2. Additionally, IL-15 is the only cytokine known to provide anti-apoptotic signaling to effector CD8⁺ T cells. IL-15, either administered alone or as a complex with the IL-15Rα, exhibits potent anti-tumor activities against well-established solid tumors in experimental animal models and, thus, has been identified as one of the most promising immunotherapeutic drugs that could potentially cure cancer.

To facilitate clinical development of an IL-15-based cancer therapeutic, an IL-15 mutant (IL-15N72D) with increased biological activity compared to IL-15 was identified (Zhu et al., J Immunol, 183: 3598-3607, 2009). The pharmacokinetics and biological activity of this IL-15 super-agonist (IL-15N72D) was further improved by the creation of IL-15N72D:IL-15RaSu/Fc fusion complex (ALT-803), such that the super agonist complex has at least 25-times the activity of the native cytokine *in vivo* (Han et al., Cytokine, 56: 804-810, 2011).

### IL-15:IL-15Rα protein complex

As described above, an IL-15:IL-15Rα fusion protein complex can refer to a complex having IL-15 non-covalently bound to the soluble IL-15Rα domain of the native IL-15Rα. In some cases, the soluble IL-15Rα is covalently linked to a biologically active polypeptide and/or to an IgG Fc domain. The IL-15 can be either IL-15 or IL-15 covalently linked to a second biologically active polypeptide. The crystal structure of the IL-15:IL-15Rα protein complex is shown in Chirifu et al., 2007 Nat Immunol 8, 1001-1007.

In various embodiments of the above aspects or any other aspect of the invention delineated herein, the IL-15Rα fusion protein comprises soluble IL-15Rα, e.g., IL-15Rα covalently linked to a biologically active polypeptide (e.g., the heavy chain constant domain of IgG, an Fc domain of the heavy chain constant domain of IgG, or a cytokine). In other embodiments of the invention of the above aspects, IL-15 comprises IL-15, e.g., IL-15 covalently linked to a second biologically active polypeptide, e.g., a cytokine. In other embodiments, purifying the IL-15:IL-15Rα fusion protein complex from the host cell or media involves capturing the IL-15:IL-15Rα fusion protein complex on an affinity reagent that specifically binds the IL-15:IL-15Rα fusion protein complex. In other embodiments, the IL-15Rα fusion protein contains an IL-15Rα/Fc fusion protein and the affinity reagent specifically binds the Fc domain. In other embodiments, the affinity reagent is Protein A or Protein G. In other embodiments, the affinity reagent is an antibody. In other embodiments, purifying the IL-15:IL-15Rα fusion protein complex from the host cell or media comprises ion exchange chromatography. In other embodiments, purifying the IL-15:IL-15Rα fusion protein complex from the host cell or media comprises size exclusion chromatography.

In other embodiments, the IL-15Rα comprises IL-15RαSushi (IL-15RaSu). In other embodiments, the IL-15 is a variant IL-15 (e.g., IL-15N72D). In other embodiments, the IL-15 binding sites of the IL-15:IL-15Rα fusion protein complex are fully occupied. In other embodiments, both IL-15 binding sites of the IL-15:IL-15RaSu/Fc fusion protein complex are fully occupied. In other embodiments, the IL-15:IL-15Rα fusion protein complex is purified based on the fusion protein complex charge or size properties. In other embodiments, the fully occupied IL-15N72D:IL-15RaSu/Fc fusion protein complex is purified by anion exchange chromatography based on the fusion protein complex charge properties. In other embodiments, the fully occupied IL-15N72D:IL-15RaSu/Fc fusion protein complex is purified using a quaternary amine-based resin with binding conditions employing low ionic strength neutral pH buffers and elution conditions employing buffers of increasing ionic strength.

In certain embodiments of the soluble fusion protein complexes of the invention, the IL-15 polypeptide is an IL-15 variant having a different amino acid sequence than native IL-15 polypeptide. The human IL-15 polypeptide is referred to herein as huIL-15, hIL-15, huIL15, hIL15, IL-15 wild type (wt) and variants thereof are referred to using the native amino acid, its position in the mature sequence and the variant amino acid. For example, huIL15N72D refers to human IL-15 comprising a substitution of N to D at position 72. In certain embodiments, the IL-15 variant functions as an IL-15 agonist as demonstrated, e.g., by increased binding activity for the IL-15RβγC receptors compared to the native IL-15 polypeptide. In certain embodiments, the IL-15 variant functions as an IL-15 antagonist as demonstrated by e.g., decreased binding activity for the IL-15RβγC receptors compared to the native IL-15 polypeptide. In certain embodiments, the IL-15 variant has increased binding affinity or a decreased binding activity for the IL-15RβγC receptors compared to the native IL-15 polypeptide. In certain embodiments, the sequence of the IL-15 variant has at least one (i.e., 1, 2, 3, 4, 5, 6, 7, 8, 9, 10, or more) amino acid change compared to the native IL-15 sequence. The amino acid change can include one or more of an amino acid substitution or deletion in the domain of IL-15 that interacts with IL-15Rβ and/or IL-15RγC. In certain embodiments, the amino acid change is one or more amino acid substitutions or deletions at position 8, 61, 65, 72, 92, 101, 108, or 111 of the mature human IL-15 sequence. For example, the amino acid change is the substitution of D to N or A at position 8, D to A at position 61, N to A at position 65, N to R at position 72 or Q to A at position 108 of the mature human IL-15 sequence, or any combination of these substitutions. In certain embodiments, the amino acid change is the substitution of N to D at position 72 of the mature human IL-15 sequence.

### Fc Domain

ALT-803 comprises an IL-15N72D:IL-15RaSu/Fc fusion complex. Fusion proteins that combine the Fc regions of IgG with the domains of another protein, such as various cytokines and soluble receptors have been reported (see, for example, Capon et al., Nature, 337:525-531, 1989; Chamow et al., Trends Biotechnol., 14:52-60, 1996); U.S. Pat. Nos. 5,116,964 and 5,541,087). The prototype fusion protein is a homodimeric protein linked through cysteine residues in the hinge region of IgG Fc, resulting in a molecule similar to an IgG molecule without the heavy chain variable and C_{H1} domains and light chains. The dimeric nature of fusion proteins comprising the Fc domain may be advantageous in providing higher order interactions (i.e. bivalent or bispecific binding) with other molecules. Due to the structural homology, Fc fusion proteins exhibit an in vivo pharmacokinetic profile comparable to that of human IgG with a similar isotype. Immunoglobulins of the IgG class are among the most abundant proteins in human blood, and their circulation half-lives can reach as long as 21 days. To extend the circulating half-life of IL-15 or an IL-15 fusion protein and/or to increase its biological activity, fusion protein complexes containing the IL-15 domain non-covalently bound to IL-15RaSu covalently linked to the Fc portion of the human heavy chain IgG protein have been made (e.g., ALT-803).

The term "Fc" refers to a non-antigen-binding fragment of an antibody. Such an "Fc" can be in monomeric or multimeric form. The original immunoglobulin source of the native Fc is preferably of human origin and may be any of the immunoglobulins, although IgG 1 and IgG2 are preferred. Native Fc's are made up of monomeric polypeptides that may be linked into dimeric or multimeric forms by covalent (i.e., disulfide bonds) and non-covalent association. The number of intermolecular disulfide bonds between monomeric subunits of native Fc molecules ranges from 1 to 4 depending on class (e.g., IgG, IgA, IgE) or subclass (e.g., IgG1, IgG2, IgG3, IgA1, IgGA2). One example of a native Fc is a disulfide-bonded dimer resulting from papain digestion of an IgG (see Ellison et al. (1982), Nucleic Acids Res. 10: 4071-9). The term "native Fc" as used herein is generic to the monomeric, dimeric, and multimeric forms. Fc domains containing binding sites for Protein A, Protein G, various Fc receptors and complement proteins.

In some embodiments, the term "Fc variant" refers to a molecule or sequence that is modified from a native Fc, but still comprises a binding site for the salvage receptor, FcRn. International applications WO 97/34631 (published Sep. 25, 1997) and WO 96/32478 describe exemplary Fc variants, as well as interaction with the salvage receptor. Thus, the term "Fc variant" comprises a molecule or sequence that is humanized from a non-human native Fc. Furthermore, a native Fc comprises sites that may be removed because they provide structural features or biological activity that are not required for the fusion molecules of the present invention. Thus, in certain embodiments, the term "Fc variant" comprises a molecule or sequence that lacks one or more native Fc sites or residues that affect or are involved in (1) disulfide bond formation, (2) incompatibility with a selected host cell (3) N-terminal heterogeneity upon expression in a selected host cell, (4) glycosylation, (5) interaction with complement, (6) binding to an Fc receptor other than a salvage receptor, (7) antibody-dependent cell-mediated cytotoxicity (ADCC), or (8) antibody dependent cellular phagocytosis (ADCP). Fc variants are described in further detail hereinafter.

The term "Fc domain" encompasses native Fc and Fc variant molecules and sequences as defined above. As with Fc variants and native Fc's, the term "Fc domain" includes molecules in monomeric or multimeric form, whether digested from whole antibody or produced by recombinant gene expression or by other means.

### Linkers

In some cases, the fusion protein complexes of the invention also include a flexible linker sequence interposed between the IL-15 or IL-15Rα domains. The linker sequence should allow effective positioning of the polypeptide with respect to the IL-15 or IL-15Rα domains to allow functional activity of both domains.

In certain cases, the soluble fusion protein complex has a linker wherein the first polypeptide is covalently linked to IL-15 (or functional fragment thereof) by a polypeptide linker sequence. In other aspects, the soluble fusion protein complex as described herein has a linker wherein the second polypeptide is covalently linked to IL-15Rα polypeptide (or functional fragment thereof) by polypeptide linker sequence.

The linker sequence is preferably encoded by a nucleotide sequence resulting in a peptide that can effectively position the binding groove of a TCR molecule for recognition of a presenting antigen or the binding domain of an antibody molecule for recognition of an antigen. As used herein, the phrase "effective positioning of the biologically active polypeptide with respect to the IL-15 or IL-15Rα domains", or other similar phrase, is intended to mean the biologically active polypeptide linked to the IL-15 or IL-15Rα domains is positioned so that the IL-15 or IL-15Rα domains are capable of interacting with each other to form a protein complex. For example, the IL-15 or IL-15Rα domains are effectively positioned to allow interactions with immune cells to initiate or inhibit an immune reaction, or to inhibit or stimulate cell development.

The fusion protein complexes of the invention preferably also include a flexible linker sequence interposed between the IL-15 or IL-15Rα domains and the immunoglobulin Fc domain. The linker sequence should allow effective positioning of the Fc domain, biologically active polypeptide and IL-15 or IL-15Rα domains to allow functional activity of each domain. For example, the Fc domains are effectively positioned to allow proper fusion protein complex formation and/or interactions with Fc receptors on immune cells or proteins of the complement system to stimulate Fc-mediated effects including opsonization, cell lysis, degranulation of mast cells, basophils, and eosinophils, and other Fc receptor-dependent processes; activation of the complement pathway; and enhanced in vivo half-life of the fusion protein complex.

Linker sequences can also be used to link two or more polypeptides of the biologically active polypeptide to generate a single-chain molecule with the desired functional activity.

Preferably, the linker sequence comprises from about 7 to 20 amino acids, more preferably from about 10 to 20 amino acids. The linker sequence is preferably flexible so as not hold the biologically active polypeptide or effector molecule in a single undesired conformation. The linker sequence can be used, e.g., to space the recognition site from the fused molecule. Specifically, the peptide linker sequence can be positioned between the biologically active polypeptide and the effector molecule, e.g., to chemically cross-link same and to provide molecular flexibility. The linker preferably predominantly comprises amino acids with small side chains, such as glycine, alanine and serine, to provide for flexibility. Preferably, about 80 or 90 percent or greater of the linker sequence comprises glycine, alanine or serine residues, particularly glycine and serine residues.

Different linker sequences could be used including any of a number of flexible linker designs that have been used successfully to join antibody variable regions together (see, Whitlow, M. et al., (1991) Methods: A Companion to Methods in Enzymology, 2:97-105).

### Fusions Protein Complexes

The invention provides ALT-803, which is a protein complex between IL-15N72D and IL-15RαSu/Fc. In certain embodiments, the ALT-803 polypeptides could serve as a scaffold for fusion to other protein domains. In such fusion protein complexes, a first fusion protein comprises a first biologically active polypeptide covalently linked to interleukin-15 (IL-15) or functional fragment thereof; and the second fusion protein comprises a second biologically active polypeptide covalently linked to soluble interleukin-15 receptor alpha (IL-15Rα) polypeptide or functional fragment thereof, where the IL-15 domain of a first fusion protein binds to the soluble IL-15Rα domain of the second fusion protein to form a soluble fusion protein complex. Fusion protein complexes of the invention also comprise immunoglobulin Fc domain or a functional fragment thereof linked to one or both of the first and second fusion proteins. Preferably, the Fc domains linked to the first and second fusion proteins interact to form a fusion protein complex. Such a complex may be stabilized by disulfide bond formation between the immunoglobulin Fc domains. In certain embodiments, the soluble fusion protein complexes of the invention include an IL-15 polypeptide, IL-15 variant or a functional fragment thereof and a soluble IL-15Rα polypeptide or a functional fragment thereof, wherein one or both of the IL-15 and IL-15Rα polypeptides further include an immunoglobulin Fc domain or a functional fragment thereof.

In a further embodiment, one or both of the first and second biologically active polypeptides comprises an antibody or functional fragment thereof.

In another embodiment, the antigen for the antibody domain comprises a cell surface receptor or ligand.

In a further embodiment, the antigen comprises a CD antigen, cytokine or chemokine receptor or ligand, growth factor receptor or ligand, tissue factor, cell adhesion molecule, MHC/MHC-like molecules, Fc receptor, Toll-like receptor, NK receptor, TCR, BCR, positive/negative co-stimulatory receptor or ligand, death receptor or ligand, tumor associated antigen, or virus encoded antigen.

As used herein, the term "biologically active polypeptide" or "effector molecule" is meant an amino acid sequence such as a protein, polypeptide or peptide; a sugar or polysaccharide; a lipid or a glycolipid, glycoprotein, or lipoprotein that can produce the desired effects as discussed herein. Effector molecules also include chemical agents. Also contemplated are effector molecule nucleic acids encoding a biologically active or effector protein, polypeptide, or peptide. Thus, suitable molecules include regulatory factors, enzymes, antibodies, or drugs as well as DNA, RNA, and oligonucleotides. The biologically active polypeptides or effector molecule can be naturally-occurring or it can be synthesized from known components, e.g., by recombinant or chemical synthesis and can include heterologous components. A biologically active polypeptides or effector molecule is generally between about 0.1 to 100 KD or greater up to about 1000 KD, preferably between about 0.1, 0.2, 0.5, 1, 2, 5, 10, 20, 30 and 50 KD as judged by standard molecule sizing techniques such as centrifugation or SDS-polyacrylamide gel electrophoresis. Desired effects include, but are not limited to, for example, forming a fusion protein complex with increased binding activity, killing a target cell, e.g. either to induce cell proliferation or cell death, initiate an immune response, in preventing or treating a disease, or to act as a detection molecule for diagnostic purposes. For such detection, an assay could be used, for example an assay that includes sequential steps of culturing cells to proliferate same.

Covalently linking the effector molecule to the fusion protein complexes disclosed herein provides a number of significant advantages. Fusion protein complexes can be produced that contain a single effector molecule, including such a peptide of known structure. Additionally, a wide variety of effector molecules can be produced in similar DNA vectors. That is, a library of different effector molecules can be linked to the fusion protein complexes for recognition of infected or diseased cells. Further, for therapeutic applications, rather than administration of a the fusion protein complex to a subject, a DNA expression vector coding for the fusion protein complex can be administered for in vivo expression of the fusion protein complex. Such an approach avoids costly purification steps typically associated with preparation of recombinant proteins and avoids the complexities of antigen uptake and processing associated with conventional approaches.

As noted, components of the fusion proteins and antibodies disclosed herein, e.g., effector molecule conjugates such as cytokines, chemokines, growth factors, protein toxins, immunoglobulin domains or other bioactive molecules and any peptide linkers, can be organized in nearly any fashion provided that the fusion protein or antibody has the function for which it was intended. In particular, each component of the fusion protein can be spaced from another component by at least one suitable peptide linker sequence if desired. Additionally, the fusion proteins may include tags, e.g., to facilitate modification, identification and/or purification of the fusion protein.

### Pharmaceutical Therapeutics

The invention provides pharmaceutical compositions comprising ALT-803 for use as a therapeutic. In one aspect, ALT-803is administered systemically, for example, formulated in a pharmaceutically-acceptable buffer such as physiological saline. Preferable routes of administration include, for example, instillation into the bladder, subcutaneous, intravenous, intraperitoneal, intramuscular, or intradermal injections that provide continuous, sustained levels of the composition in the patient. Treatment of human patients or other animals is carried out using a therapeutically effective amount of a therapeutic identified herein in a physiologically-acceptable carrier. Suitable carriers and their formulation are described, for example, in Remington's Pharmaceutical Sciences by E. W. Martin. The amount of the therapeutic agent to be administered varies depending upon the manner of administration, the age and body weight of the patient, and with the clinical symptoms of the neoplasia. Generally, amounts will be in the range of those used for other agents used in the treatment of other diseases associated with neoplasia, although in certain instances lower amounts will be needed because of the increased specificity of the compound. A compound is administered at a dosage that enhances an immune response of a subject, or that reduces the proliferation, survival, or invasiveness of a neoplastic cell as determined by a method known to one skilled in the art.

### Formulation of Pharmaceutical Compositions

The administration of ALT-803 for the treatment of a neoplasia may be by any suitable means that results in a concentration of the therapeutic that, combined with other components, is effective in ameliorating, reducing, or stabilizing a neoplasia. ALT-803 may be contained in any appropriate amount in any suitable carrier substance, and is generally present in an amount of 1-95% by weight of the total weight of the composition. The composition may be provided in a dosage form that is suitable for parenteral (e.g., subcutaneously, intravenously, intramuscularly, intravesicularly or intraperitoneally) administration route. The pharmaceutical compositions may be formulated according to conventional pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York).

Human dosage amounts can initially be determined by extrapolating from the amount of compound used in mice or nonhuman primates, as a skilled artisan recognizes it is routine in the art to modify the dosage for humans compared to animal models. In certain embodiments it is envisioned that the dosage may vary from between about 0.1 µg compound/kg body weight to about 5000 µg compound/kg body weight; or from about 1 µg/kg body weight to about 4000 µg/kg body weight or from about 10 µg/kg body weight to about 3000 µg/kg body weight. In other embodiments this dose may be about 0.1, 0.3, 0.5, 1, 3, 5, 10, 25, 50, 75, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1600, 1700, 1800, 1900, 2000, 2500, 3000, 3500, 4000, 4500, or 5000 µg/kg body weight. In other embodiments, it is envisaged that doses may be in the range of about 0.5 µg compound/kg body weight to about 20 µg compound/kg body weight. In other embodiments the doses may be about 0.5, 1, 3, 6, 10, or 20 mg/kg body weight. Of course, this dosage amount may be adjusted upward or downward, as is routinely done in such treatment protocols, depending on the results of the initial clinical trials and the needs of a particular patient.

In particular embodiments, ALT-803 are formulated in an excipient suitable for parenteral administration. In particular embodiments, ALT-803 is administered at 0.5 µg/kg-about 15 µg/kg (e.g., 0.5, 1, 3, 5, 10, or 15 µg/kg).

For the treatment of bladder cancer, ALT-803 is administered by instillation into the bladder. Methods of instillation are known. See, for example, Lawrencia, et al., Gene Ther 8, 760-8 (2001); Nogawa, et al., J Clin Invest 115, 978-85 (2005); Ng, et al., Methods Enzymol 391, 304-13 2005; Tyagi, et al., J Urol 171, 483-9 (2004); Trevisani, et al., J Pharmacol Exp Ther 309, 1167-73 (2004); Trevisani, et al., Nat Neurosci 5, 546-51 (2002)); (Segal, et al., 1975). (Dyson, et al., 2005). (Batista, et al., 2005; Dyson, et al., 2005). In certain embodiments, it is envisioned that the ALT-803 dosage for instillation may vary from between about 5 and 1000 µg/dose.

Pharmaceutical compositions are formulated with appropriate excipients into a pharmaceutical composition that, upon administration, releases the therapeutic in a controlled manner. Examples include single or multiple unit tablet or capsule compositions, oil solutions, suspensions, emulsions, microcapsules, microspheres, molecular complexes, nanoparticles, patches, and liposomes.

### Parenteral Compositions

The pharmaceutical composition comprising ALT-803 may be administered parenterally by injection, infusion or implantation (subcutaneous, intravenous, intramuscular, intravesicularly, intraperitoneal, or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation. Formulations can be found in Remington: The Science and Practice of Pharmacy, supra.

Compositions comprising ALT-803 for parenteral use may be provided in unit dosage forms (e.g., in single-dose ampoules, syringes or bags), or in vials containing several doses and in which a suitable preservative may be added (see below). The composition may be in the form of a solution, a suspension, an emulsion, an infusion device, or a delivery device for implantation, or it may be presented as a dry powder to be reconstituted with water or another suitable vehicle before use. Apart from the active agent that reduces or ameliorates a neoplasia, the composition may include suitable parenterally acceptable carriers and/or excipients. The active therapeutic agent(s) may be incorporated into microspheres, microcapsules, nanoparticles, liposomes, or the like for controlled release. Furthermore, the composition may include suspending, solubilizing, stabilizing, pH-adjusting agents, tonicity adjusting agents, and/or dispersing, agents.

As indicated above, the pharmaceutical compositions comprising ALT-803 may be in a form suitable for sterile injection. To prepare such a composition, the suitable active antineoplastic/anti-infective therapeutic(s) are dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution, and isotonic sodium chloride solution and dextrose solution. The aqueous formulation may also contain one or more preservatives (e.g., methyl, ethyl or n-propyl p-hydroxybenzoate). In cases where one of the compounds is only sparingly or slightly soluble in water, a dissolution enhancing or solubilizing agent can be added, or the solvent may include 10-60% w/w of propylene glycol or the like.

The present invention provides combinations for use in methods of treating neoplastic disorders or symptoms thereof which comprise administering a therapeutically effective amount of a pharmaceutical composition comprising a compound of the formulae herein to a subject (e.g., a mammal such as a human). Thus, one embodiment is a method of treating a subject suffering from or susceptible to a neoplastic disease or disorder or symptom thereof. The method includes the step of administering to the mammal a therapeutic amount of an amount of a compound herein sufficient to treat the disease or disorder or symptom thereof, under conditions such that the disease or disorder is treated.

The methods herein include administering to the subject (including a subject identified as in need of such treatment) an effective amount of a compound described herein, or a composition described herein to produce such effect. Identifying a subject in need of such treatment can be in the judgment of a subject or a health care professional and can be subjective (e.g. opinion) or objective (e.g. measurable by a test or diagnostic method).

The therapeutic methods of the invention (which include prophylactic treatment) in general comprise administration of a therapeutically effective amount of the compounds herein, such as a compound of the formulae herein to a subject (e.g., animal, human) in need thereof, including a mammal, particularly a human. Such treatment will be suitably administered to subjects, particularly humans, suffering from, having, susceptible to, or at risk for a neoplastic or infectious disease, disorder, or symptom thereof. Determination of those subjects "at risk" can be made by any objective or subjective determination by a diagnostic test or opinion of a subject or health care provider (e.g., genetic test, enzyme or protein marker, Marker (as defined herein), family history, and the like).

In one embodiment, the disclosure provides a method of monitoring treatment progress. The method includes the step of determining a level of diagnostic marker (Marker) (e.g., any target delineated herein modulated by a compound herein, a protein or indicator thereof, etc.) or diagnostic measurement (e.g., screen, assay) in a subject suffering from or susceptible to a disorder or symptoms thereof associated with neoplasia in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

### Combination Therapies

Preferably, ALT-803 is administered in combination with an anti-neoplasia therapeutic such as an antibody, e.g., a tumor-specific antibody or an immune-checkpoint inhibitor. The antibody and ALT-803 may be administered simultaneously or sequentially. In some embodiments, the antibody treatment is an established therapy for the disease indication and addition of ALT-803 treatment to the antibody regimen improves the therapeutic benefit to the patients. Such improvement could be measured as increased responses on a per patient basis or increased responses in the patient population. Combination therapy could also provide improved responses at lower or less frequent doses of antibody resulting in a better tolerated treatment regimen. As indicated, the combined therapy of ALT-803 and an antibody could provide enhances clinical activity through various mechanisms, including augmented ADCC, ADCP, and/or NK cell, T-cell, neutrophil or monocytic cell levels or immune responses.

If desired, ALT-803 is administered in combination with any conventional therapy, including but not limited to, surgery, radiation therapy, chemotherapy, protein-based therapy or biological therapy. Chemotherapeutic drugs include alkylating agents (e.g., platinum-based drugs, tetrazines, aziridines, nitrosoureas, nitrogen mustards), anti-metabolites (e.g., anti-folates, fluoropyrimidines, deoxynucleoside analogues, thiopurines), anti-microtubule agents (e.g., vinca alkaloids, taxanes), topoisomerase inhibitors (e.g., topoisomerase I and II inhibitors), cytotoxic antibiotics (e.g., anthracyclines) and immunomodulatory drugs (e.g., thalidomide and analogs).

### Kits or Pharmaceutical Systems

Pharmaceutical compositions comprising ALT-803 may be assembled into kits or pharmaceutical systems for use in treating a neoplasia. Kits or pharmaceutical systems according to this aspect of the invention comprise a carrier means, such as a box, carton, tube, having in close confinement therein one or more container means, such as vials, tubes, ampoules, bottles, syringes, or bags. The kits or pharmaceutical systems of the invention may also comprise associated instructions for using ALT-803.

### Recombinant Protein Expression

In general, preparation of the fusion protein complexes of the invention (e.g., components of ALT-803) can be accomplished by procedures disclosed herein and by recognized recombinant DNA techniques.

In general, recombinant polypeptides are produced by transformation of a suitable host cell with all or part of a polypeptide-encoding nucleic acid molecule or fragment thereof in a suitable expression vehicle. Those skilled in the field of molecular biology will understand that any of a wide variety of expression systems may be used to provide the recombinant protein. The precise host cell used is not critical to the invention. A recombinant polypeptide may be produced in virtually any eukaryotic host (e.g., *Saccharomyces cerevisiae,* insect cells, e.g., Sf21 cells, or mammalian cells, e.g., NIH 3T3, HeLa, COS or preferably CHO cells). Such cells are available from a wide range of sources (e.g., the American Type Culture Collection, Rockland, Md.; also, see, e.g., Ausubel et al., Current Protocol in Molecular Biology, New York: John Wiley and Sons, 1997). The method of transfection and the choice of expression vehicle will depend on the host system selected. Transformation methods are described, e.g., in Ausubel et al. (supra); expression vehicles may be chosen from those provided, e.g., in Cloning Vectors: A Laboratory Manual (P. H. Pouwels et al., 1985, Supp. 1987).

A variety of expression systems exist for the production of recombinant polypeptides. Expression vectors useful for producing such polypeptides include, without limitation, chromosomal, episomal, and virus-derived vectors, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof.

Once the recombinant polypeptide is expressed, it is isolated, e.g., using affinity chromatography. In one example, an antibody (e.g., produced as described herein) raised against the polypeptide may be attached to a column and used to isolate the recombinant polypeptide. Lysis and fractionation of polypeptide-harboring cells prior to affinity chromatography may be performed by standard methods (see, e.g., Ausubel et al., supra). Once isolated, the recombinant protein can, if desired, be further purified, e.g., by high performance liquid chromatography (see, e.g., Fisher, Laboratory Techniques In Biochemistry and Molecular Biology, eds., Work and Burdon, Elsevier, 1980).

As used herein, biologically active polypeptides or effector molecules of the invention may include factors such as cytokines, chemokines, growth factors, protein toxins, immunoglobulin domains or other bioactive proteins such as enzymes. Also biologically active polypeptides may include conjugates to other compounds such as non-protein toxins, cytotoxic agents, chemotherapeutic agents, detectable labels, radioactive materials and such.

Cytokines of the invention are defined by any factor produced by cells that affect other cells and are responsible for any of a number of multiple effects of cellular immunity. Examples of cytokines include but are not limited to the IL-2 family, interferon (IFN), IL-10, IL-1, IL-17, TGF and TNF cytokine families, and to IL-1 through IL-35, IFN-α, IFN-β, IFNγ, TGF-β, TNF-α, and TNFβ.

In an aspect of the invention, the first fusion protein comprises a first biologically active polypeptide covalently linked to interleukin-15 (IL-15) domain or a functional fragment thereof. IL-15 is a cytokine that affects T-cell activation and proliferation. IL-15 activity in affecting immune cell activation and proliferation is similar in some respects to IL-2, although fundamental differences have been well characterized (Waldmann, T A, 2006, Nature Rev. Immunol. 6:595-601).

In another aspect of the invention, the first fusion protein comprises an interleukin-15 (IL-15) domain that is an IL-15 variant (also referred to herein as IL-15 mutant). The IL-15 variant preferably comprises a different amino acid sequence that the native (or wild type) IL-15 protein. The IL-15 variant preferably binds the IL-15Rα polypeptide and functions as an IL-15 agonist or antagonist. Preferably, IL-15 variants with agonist activity have super agonist activity. In some embodiments, the IL-15 variant can function as an IL-15 agonist or antagonist independent of its association with IL-15Rα. IL-15 agonists are exemplified by comparable or increased biological activity compared to wild type IL-15. IL-15 antagonists are exemplified by decreased biological activity compared to wild type IL-15 or by the ability to inhibit IL-15-mediated responses. In some examples, the IL-15 variant binds with increased or decreased activity to the IL-15RβγC receptors. In some embodiments, the sequence of the IL-15 variant has at least one amino acid change, e.g. substitution or deletion, compared to the native IL-15 sequence, such changes resulting in IL-15 agonist or antagonist activity. Preferably the amino acid substitutions/deletions are in the domains of IL-15 that interact with IL-15Rβ and/or γC. More preferably, the amino acid substitutions/deletions do not affect binding to the IL-15Rα polypeptide or the ability to produce the IL-15 variant. Suitable amino acid substitutions/deletions to generate IL-15 variants can be identified based on putative or known IL-15 structures, comparisons of IL-15 with homologous molecules such as IL-2 with known structure, through rational or random mutagenesis and functional assays, as provided herein, or other empirical methods. Additionally suitable amino acid substitutions can be conservative or non-conservative changes and insertions of additional amino acids. Preferably, IL-15 variants of the invention contain one or more than one amino acid substitutions/deletions at position 6, 8, 10, 61, 65, 72, 92, 101, 104, 105, 108, 109, 111, or 112 of the mature human IL-15 sequence; particularly, D8N ("D8" refers to the amino acid and residue position in the native mature human IL-15 sequence and "N" refers to the substituted amino acid residue at that position in the IL-15 variant), I6S, D8A, D61A, N65A, N72R, V104P or Q108A substitutions result in IL-15 variants with antagonist activity and N72D substitutions result in IL-15 variants with agonist activity.

Chemokines, similar to cytokines, are defined as any chemical factor or molecule which when exposed to other cells are responsible for any of a number of multiple effects of cellular immunity. Suitable chemokines may include but are not limited to the CXC, CC, C, and CX.sub.3C chemokine families and to CCL-1 through CCL-28, CXC-1 through CXC-17, XCL-1, XCL-2, CX3CL1, MIP-1b, IL-8, MCP-1, and Rantes.

Growth factors include any molecules which when exposed to a particular cell induce proliferation and/or differentiation of the affected cell. Growth factors include proteins and chemical molecules, some of which include: GM-CSF, G-CSF, human growth factor and stem cell growth factor. Additional growth factors may also be suitable for uses described herein.

Toxins or cytotoxic agents include any substance that has a lethal effect or an inhibitory effect on growth when exposed to cells. More specifically, the effector molecule can be a cell toxin of, e.g., plant or bacterial origin such as, e.g., diphtheria toxin (DT), shiga toxin, abrin, cholera toxin, ricin, saporin, pseudomonas exotoxin (PE), pokeweed antiviral protein, or gelonin. Biologically active fragments of such toxins are well known in the art and include, e.g., DT A chain and ricin A chain. Additionally, the toxin can be an agent active at the cell surface such as, e.g., phospholipase enzymes (e.g., phospholipase C).

Further, the effector molecule can be a chemotherapeutic drug such as, e.g., vindesine, vincristine, vinblastin, methotrexate, adriamycin, bleomycin, or cisplatin.

Additionally, the effector molecule can be a detectably-labeled molecule suitable for diagnostic or imaging studies. Such labels include biotin or streptavidin/avidin, a detectable nanoparticles or crystal, an enzyme or catalytically active fragment thereof, a fluorescent label such as green fluorescent protein, FITC, phycoerythrin, cychome, Texas red or quantum dots; a radionuclide e.g., iodine-131, yttrium-90, rhenium-188 or bismuth-212; a phosphorescent or chemiluminescent molecules or a label detectable by PET, ultrasound or MRI such as Gd- or paramagnetic metal ion-based contrast agents. See e.g., Moskaug, et al. J. Biol. Chem. 264, 15709 (1989); Pastan, I. et al. Cell 47, 641, 1986; Pastan et al., Recombinant Toxins as Novel Therapeutic Agents, Ann. Rev. Biochem. 61, 331, (1992); "Chimeric Toxins" Olsnes and Phil, Pharmac. Ther., 25, 355 (1982); published PCT application no. WO 94/29350; published PCT application no. WO 94/04689; published PCT application no. WO2005046449 and U.S. Pat. No. 5,620,939 for disclosure relating to making and using proteins comprising effectors or tags.

A protein fusion or conjugate complex that includes a covalently linked IL-15 and IL-15Rα domains has several important uses. Cells or tissue susceptible to being damaged or killed can be readily assayed by the methods disclosed herein.

The IL-15 and IL-15Rα polypeptides of the invention suitably correspond in amino acid sequence to naturally occurring IL-15 and IL-15Rα molecules, e.g. IL-15 and IL-15Rα molecule of a human, mouse or other rodent, or other mammal. Sequences of these polypeptides and encoding nucleic acids are known in the literature, including human interleukin 15 (IL15) mRNA--GenBank: U14407.1, Mus musculus interleukin 15 (IL15) mRNA--GenBank: U14332.1, human interleukin-15 receptor alpha chain precursor (IL15RA) mRNA--GenBank: U31628.1, Mus musculus interleukin 15 receptor, alpha chain--GenBank: BC095982.1.

In some settings, it can be useful to make the protein fusion or conjugate complexes of the present invention polyvalent, e.g., to increase the valency of the sc-TCR or sc-antibody. In particular, interactions between the IL-15 and IL-15Rα domains of the fusion protein complex provide a means of generating polyvalent complexes. In addition, the polyvalent fusion protein can made by covalently or non-covalently linking together between one and four proteins (the same or different) by using e.g., standard biotin-streptavidin labeling techniques, or by conjugation to suitable solid supports such as latex beads. Chemically cross-linked proteins (for example cross-linked to nanoparticles) are also suitable polyvalent species. For example, the protein can be modified by including sequences encoding tag sequences that can be modified such as the biotinylation BirA tag or amino acid residues with chemically reactive side chains such as Cys or His. Such amino acid tags or chemically reactive amino acids may be positioned in a variety of positions in the fusion protein or antibody, preferably distal to the active site of the biologically active polypeptide or effector molecule. For example, the C-terminus of a soluble fusion protein can be covalently linked to a tag or other fused protein which includes such a reactive amino acid(s). Suitable side chains can be included to chemically link two or more fusion proteins to a suitable nanoparticle to give a multivalent molecule. Exemplary nanoparticles include dendrimers, liposomes, core-shell particles or PLGA-based particles.

In another embodiment of the invention, one or both of the polypeptides of the fusion protein complex comprises an immunoglobulin domain. Alternatively, the protein binding domain-IL-15 fusion protein can be further linked to an immunoglobulin domain. The preferred immunoglobulin domains comprise regions that allow interaction with other immunoglobulin domains to form multichain proteins as provided above. For example, the immunoglobulin heavy chain regions, such as the IgG1 C_{H2}-C_{H3}, are capable of stably interacting to create the Fc region. Preferred immunoglobulin domains including Fc domains also comprise regions with effector functions, including Fc receptor or complement protein binding activity, and/or with glycosylation sites. In some embodiments, the immunoglobulin domains of the fusion protein complex contain mutations that reduce or augment Fc receptor or complement binding activity or glycosylation, thereby affecting the biological activity of the resulting protein. For example, immunoglobulin domains containing mutations that reduce binding to Fc receptors could be used to generate fusion protein complex of the invention with lower binding activity to Fc receptor-bearing cells, which may be advantageous for reagents designed to recognize or detect specific antigens.

### Nucleic Acids and Vectors

The disclosure further provides nucleic acid sequences and particularly DNA sequences that encode the present proteins (e.g., components of ALT-803). Preferably, the DNA sequence is carried by a vector suited for extrachromosomal replication such as a phage, virus, plasmid, phagemid, cosmid, YAC, or episome. In particular, a DNA vector that encodes a desired fusion protein can be used to facilitate preparative methods described herein and to obtain significant quantities of the fusion protein. The DNA sequence can be inserted into an appropriate expression vector, i.e., a vector that contains the necessary elements for the transcription and translation of the inserted protein-coding sequence. A variety of host-vector systems may be utilized to express the protein-coding sequence. These include mammalian cell systems infected with virus (e.g., vaccinia virus, adenovirus, etc.); insect cell systems infected with virus (e.g., baculovirus); microorganisms such as yeast containing yeast vectors, or bacteria transformed with bacteriophage DNA, plasmid DNA or cosmid DNA. Depending on the host-vector system utilized, any one of a number of suitable transcription and translation elements may be used. See, Sambrook et al., supra and Ausubel et al. supra.

Disclosed herein are methods for making a soluble fusion protein complex, the method comprising introducing into a host cell a DNA vector as described herein encoding the first and second fusion proteins, culturing the host cell in media under conditions sufficient to express the fusion proteins in the cell or the media and allow association between IL-15 domain of a first fusion protein and the soluble IL-15Rα domain of a second fusion protein to form the soluble fusion protein complex, purifying the soluble fusion protein complex from the host cells or media.

In general, a preferred DNA vector disclosed herein comprises a nucleotide sequence linked by phosphodiester bonds comprising, in a 5' to 3' direction a first cloning site for introduction of a first nucleotide sequence encoding a biologically active polypeptide, operatively linked to a sequence encoding an effector molecule.

The fusion protein components encoded by the DNA vector can be provided in a cassette format. By the term "cassette" is meant that each component can be readily substituted for another component by standard recombinant methods. In particular, a DNA vector configured in a cassette format is particularly desirable when the encoded fusion complex is to be used against pathogens that may have or have capacity to develop serotypes.

To make the vector coding for a fusion protein complex, the sequence coding for the biologically active polypeptide is linked to a sequence coding for the effector peptide by use of suitable ligases. DNA coding for the presenting peptide can be obtained by isolating DNA from natural sources such as from a suitable cell line or by known synthetic methods, e.g. the phosphate triester method. See, e.g., Oligonucleotide Synthesis, IRL Press (M. J. Gait, ed., 1984). Synthetic oligonucleotides also may be prepared using commercially available automated oligonucleotide synthesizers. Once isolated, the gene coding for the biologically active polypeptide can be amplified by the polymerase chain reaction (PCR) or other means known in the art. Suitable PCR primers to amplify the biologically active polypeptide gene may add restriction sites to the PCR product. The PCR product preferably includes splice sites for the effector peptide and leader sequences necessary for proper expression and secretion of the biologically active polypeptide -effector fusion complex. The PCR product also preferably includes a sequence coding for the linker sequence, or a restriction enzyme site for ligation of such a sequence.

The fusion proteins described herein are preferably produced by standard recombinant DNA techniques. For example, once a DNA molecule encoding the biologically active polypeptide is isolated, sequence can be ligated to another DNA molecule encoding the effector polypeptide. The nucleotide sequence coding for a biologically active polypeptide may be directly joined to a DNA sequence coding for the effector peptide or, more typically, a DNA sequence coding for the linker sequence as discussed herein may be interposed between the sequence coding for the biologically active polypeptide and the sequence coding for the effector peptide and joined using suitable ligases. The resultant hybrid DNA molecule can be expressed in a suitable host cell to produce the fusion protein complex. The DNA molecules are ligated to each other in a 5' to 3' orientation such that, after ligation, the translational frame of the encoded polypeptides is not altered (i.e., the DNA molecules are ligated to each other in-frame). The resulting DNA molecules encode an in-frame fusion protein.

Other nucleotide sequences also can be included in the gene construct. For example, a promoter sequence, which controls expression of the sequence coding for the biologically active polypeptide fused to the effector peptide, or a leader sequence, which directs the fusion protein to the cell surface or the culture medium, can be included in the construct or present in the expression vector into which the construct is inserted. An immunoglobulin or CMV promoter is particularly preferred.

In obtaining variant biologically active polypeptide, IL-15, IL-15Rα or Fc domain coding sequences, those of ordinary skill in the art will recognize that the polypeptides may be modified by certain amino acid substitutions, additions, deletions, and post-translational modifications, without loss or reduction of biological activity. In particular, it is well-known that conservative amino acid substitutions, that is, substitution of one amino acid for another amino acid of similar size, charge, polarity and conformation, are unlikely to significantly alter protein function. The 20 standard amino acids that are the constituents of proteins can be broadly categorized into four groups of conservative amino acids as follows: the nonpolar (hydrophobic) group includes alanine, isoleucine, leucine, methionine, phenylalanine, proline, tryptophan and valine; the polar (uncharged, neutral) group includes asparagine, cysteine, glutamine, glycine, serine, threonine and tyrosine; the positively charged (basic) group contains arginine, histidine and lysine; and the negatively charged (acidic) group contains aspartic acid and glutamic acid. Substitution in a protein of one amino acid for another within the same group is unlikely to have an adverse effect on the biological activity of the protein. In other instance, modifications to amino acid positions can be made to reduce or enhance the biological activity of the protein. Such changes can be introduced randomly or via site-specific mutations based on known or presumed structural or functional properties of targeted residue(s). Following expression of the variant protein, the changes in the biological activity due to the modification can be readily assessed using binding or functional assays.

Homology between nucleotide sequences can be determined by DNA hybridization analysis, wherein the stability of the double-stranded DNA hybrid is dependent on the extent of base pairing that occurs. Conditions of high temperature and/or low salt content reduce the stability of the hybrid, and can be varied to prevent annealing of sequences having less than a selected degree of homology. For instance, for sequences with about 55% G-C content, hybridization and wash conditions of 40-50 °C, 6 × SSC (sodium chloride/sodium citrate buffer) and 0.1% SDS (sodium dodecyl sulfate) indicate about 60-70% homology, hybridization and wash conditions of 50-65 °C, 1 × SSC and 0.1% SDS indicate about 82-97% homology, and hybridization and wash conditions of 52 °C, 0.1 × SSC and 0.1% SDS indicate about 99-100% homology. A wide range of computer programs for comparing nucleotide and amino acid sequences (and measuring the degree of homology) are also available, and a list providing sources of both commercially available and free software is found in Ausubel et al. (1999). Readily available sequence comparison and multiple sequence alignment algorithms are, respectively, the Basic Local Alignment Search Tool (BLAST) (Altschul et al., 1997) and ClustalW programs. BLAST is available on the world wide web at ncbi.nlm.nih.gov and a version of ClustalW is available at 2.ebi.ac.uk.

The components of the fusion protein can be organized in nearly any order provided each is capable of performing its intended function. For example, in one embodiment, the biologically active polypeptide is situated at the C or N terminal end of the effector molecule.

Preferred effector molecules of the invention will have sizes conducive to the function for which those domains are intended. The effector molecules of the invention can be made and fused to the biologically active polypeptide by a variety of methods including well-known chemical cross-linking methods. See, e.g., Means, G. E. and Feeney, R. E. (1974) in Chemical Modification of Proteins, Holden-Day. See also, S. S. Wong (1991) in Chemistry of Protein Conjugation and Cross-Linking, CRC Press. However it is generally preferred to use recombinant manipulations to make the in-frame fusion protein.

As noted, a fusion molecule or a conjugate molecule in accord with the invention can be organized in several ways. In an exemplary configuration, the C-terminus of the biologically active polypeptide is operatively linked to the N-terminus of the effector molecule. That linkage can be achieved by recombinant methods if desired. However, in another configuration, the N-terminus of the biologically active polypeptide is linked to the C-terminus of the effector molecule.

Alternatively, or in addition, one or more additional effector molecules can be inserted into the biologically active polypeptide or conjugate complexes as needed.

### Vectors and Expression

A number of strategies can be employed to express ALT-803. For example, a construct encoding ALT-803 can be incorporated into a suitable vector using restriction enzymes to make cuts in the vector for insertion of the construct followed by ligation. The vector containing the gene construct is then introduced into a suitable host for expression of the fusion protein. See, generally, Sambrook et al., *supra.* Selection of suitable vectors can be made empirically based on factors relating to the cloning protocol. For example, the vector should be compatible with, and have the proper replicon for the host that is being employed. The vector must be able to accommodate the DNA sequence coding for the fusion protein complex that is to be expressed. Suitable host cells include eukaryotic and prokaryotic cells, preferably those cells that can be easily transformed and exhibit rapid growth in culture medium. Specifically preferred hosts cells include prokaryotes such as *E. coli, Bacillus subtillus,* etc. and eukaryotes such as animal cells and yeast strains, e.g., *S. cerevisiae.* Mammalian cells are generally preferred, particularly J558, NSO, SP2-O or CHO. Other suitable hosts include, e.g., insect cells such as Sf9. Conventional culturing conditions are employed. See, Sambrook, supra. Stable transformed or transfected cell lines can then be selected. Cells expressing a fusion protein complex of the invention can be determined by known procedures. For example, expression of a fusion protein complex linked to an immunoglobulin can be determined by an ELISA specific for the linked immunoglobulin and/or by immunoblotting. Other methods for detecting expression of fusion proteins comprising biologically active polypeptides linked to IL-15 or IL-15Rα domains are disclosed in the Examples.

As mentioned generally above, a host cell can be used for preparative purposes to propagate nucleic acid encoding a desired fusion protein. Thus, a host cell can include a prokaryotic or eukaryotic cell in which production of the fusion protein is specifically intended. Thus host cells specifically include yeast, fly, worm, plant, frog, mammalian cells and organs that are capable of propagating nucleic acid encoding the fusion. Non-limiting examples of mammalian cell lines which can be used include CHO dhfr-cells (Urlaub and Chasm, Proc. Natl. Acad. Sci. USA, 77:4216 (1980)), 293 cells (Graham et al., J Gen. Virol., 36:59 (1977)) or myeloma cells like SP2 or NSO (Galfre and Milstein, Meth. Enzymol., 73(B):3 (1981)).

Host cells capable of propagating nucleic acid encoding a desired fusion protein complexes encompass non-mammalian eukaryotic cells as well, including insect (e.g., Sp. *frugiperda),* yeast (e.g., *S. cerevisiae, S. pombe, P. pastoris., K. lactis, H. polymorpha;* as generally reviewed by Fleer, R., Current Opinion in Biotechnology, 3(5):486496 (1992)), fungal and plant cells. Also contemplated are certain prokaryotes such as *E. coli* and *Bacillus.*

Nucleic acid encoding a desired fusion protein can be introduced into a host cell by standard techniques for transfecting cells. The term "transfecting" or "transfection" is intended to encompass all conventional techniques for introducing nucleic acid into host cells, including calcium phosphate co-precipitation, DEAE-dextran-mediated transfection, lipofection, electroporation, microinjection, viral transduction and/or integration. Suitable methods for transfecting host cells can be found in Sambrook et al. supra, and other laboratory textbooks.

Various promoters (transcriptional initiation regulatory region) may be used. The selection of the appropriate promoter is dependent upon the proposed expression host. Promoters from heterologous sources may be used as long as they are functional in the chosen host.

Promoter selection is also dependent upon the desired efficiency and level of peptide or protein production. Inducible promoters such as tac are often employed in order to dramatically increase the level of protein expression in *E. coli.* Overexpression of proteins may be harmful to the host cells. Consequently, host cell growth may be limited. The use of inducible promoter systems allows the host cells to be cultivated to acceptable densities prior to induction of gene expression, thereby facilitating higher product yields.

Various signal sequences may be used. A signal sequence which is homologous to the biologically active polypeptide coding sequence may be used. Alternatively, a signal sequence which has been selected or designed for efficient secretion and processing in the expression host may also be used. For example, suitable signal sequence/host cell pairs include the *B. subtilis* sacB signal sequence for secretion in *B. subtilis,* and the *Saccharomyces cerevisiae* α-mating factor or *P. pastoris* acid phosphatase phoI signal sequences for *P*. *pastoris* secretion. The signal sequence may be joined directly through the sequence encoding the signal peptidase cleavage site to the protein coding sequence, or through a short nucleotide bridge consisting of usually fewer than ten codons, where the bridge ensures correct reading frame of the downstream protein sequence.

Elements for enhancing transcription and translation have been identified for eukaryotic protein expression systems. For example, positioning the cauliflower mosaic virus (CaMV) promoter 1000 bp on either side of a heterologous promoter may elevate transcriptional levels by 10- to 400-fold in plant cells. The expression construct should also include the appropriate translational initiation sequences. Modification of the expression construct to include a Kozak consensus sequence for proper translational initiation may increase the level of translation by 10 fold.

A selective marker is often employed, which may be part of the expression construct or separate from it (e.g., carried by the expression vector), so that the marker may integrate at a site different from the gene of interest. Examples include markers that confer resistance to antibiotics (e.g., bla confers resistance to ampicillin for *E. coli* host cells, nptII confers kanamycin resistance to a wide variety of prokaryotic and eukaryotic cells) or that permit the host to grow on minimal medium (e.g., HIS4 enables *P. pastoris* or His⁻*S*. *cerevisiae* to grow in the absence of histidine). The selectable marker has its own transcriptional and translational initiation and termination regulatory regions to allow for independent expression of the marker. If antibiotic resistance is employed as a marker, the concentration of the antibiotic for selection will vary depending upon the antibiotic, generally ranging from 10 to 600 µg of the antibiotic/mL of medium.

The expression construct is assembled by employing known recombinant DNA techniques (Sambrook et al., 1989; Ausubel et al., 1999). Restriction enzyme digestion and ligation are the basic steps employed to join two fragments of DNA. The ends of the DNA fragment may require modification prior to ligation, and this may be accomplished by filling in overhangs, deleting terminal portions of the fragment(s) with nucleases (e.g., ExoIII), site directed mutagenesis, or by adding new base pairs by PCR. Polylinkers and adaptors may be employed to facilitate joining of selected fragments. The expression construct is typically assembled in stages employing rounds of restriction, ligation, and transformation of *E. coli.* Numerous cloning vectors suitable for construction of the expression construct are known in the art (λZAP and pBLUESCRIPT SK-1, Stratagene, La Jolla, CA, pET, Novagen Inc., Madison, WI, cited in Ausubel et al., 1999) and the particular choice is not critical to the invention. The selection of cloning vector will be influenced by the gene transfer system selected for introduction of the expression construct into the host cell. At the end of each stage, the resulting construct may be analyzed by restriction, DNA sequence, hybridization and PCR analyses.

The expression construct may be transformed into the host as the cloning vector construct, either linear or circular, or may be removed from the cloning vector and used as is or introduced onto a delivery vector. The delivery vector facilitates the introduction and maintenance of the expression construct in the selected host cell type. The expression construct is introduced into the host cells by any of a number of known gene transfer systems (e.g., natural competence, chemically mediated transformation, protoplast transformation, electroporation, biolistic transformation, transfection, or conjugation) (Ausubel et al., 1999; Sambrook et al., 1989). The gene transfer system selected depends upon the host cells and vector systems used.

For instance, the expression construct can be introduced into *S. cerevisiae* cells by protoplast transformation or electroporation. Electroporation of *S. cerevisiae* is readily accomplished, and yields transformation efficiencies comparable to spheroplast transformation.

The present disclosure further provides a production process for isolating a fusion protein of interest. In the process, a host cell (e.g., a yeast, fungus, insect, bacterial or animal cell), into which has been introduced a nucleic acid encoding the protein of the interest operatively linked to a regulatory sequence, is grown at production scale in a culture medium to stimulate transcription of the nucleotides sequence encoding the fusion protein of interest. Subsequently, the fusion protein of interest is isolated from harvested host cells or from the culture medium. Standard protein purification techniques can be used to isolate the protein of interest from the medium or from the harvested cells. In particular, the purification techniques can be used to express and purify a desired fusion protein on a large-scale (i.e. in at least milligram quantities) from a variety of implementations including roller bottles, spinner flasks, tissue culture plates, bioreactor, or a fermenter.

An expressed protein fusion complex can be isolated and purified by known methods. Typically the culture medium is centrifuged or filtered and then the supernatant is purified by affinity or immunoaffinity chromatography, e.g. Protein-A or Protein-G affinity chromatography or an immunoaffinity protocol comprising use of monoclonal antibodies that bind the expressed fusion complex. The fusion proteins of the present invention can be separated and purified by appropriate combination of known techniques. These methods include, for example, methods utilizing solubility such as salt precipitation and solvent precipitation, methods utilizing the difference in molecular weight such as dialysis, ultrafiltration, gel-filtration, and SDS-polyacrylamide gel electrophoresis, methods utilizing a difference in electrical charge such as ion-exchange column chromatography, methods utilizing specific affinity such as affinity chromatography, methods utilizing a difference in hydrophobicity such as reverse-phase high performance liquid chromatography and methods utilizing a difference in isoelectric point, such as isoelectric focusing electrophoresis, metal affinity columns such as Ni-NTA. See generally Sambrook et al. and Ausubel et al. supra for disclosure relating to these methods.

It is preferred that the fusion proteins of the present invention be substantially pure. That is, the fusion proteins have been isolated from cell substituents that naturally accompany it so that the fusion proteins are present preferably in at least 80% or 90% to 95% homogeneity (w/w). Fusion proteins having at least 98 to 99% homogeneity (w/w) are most preferred for many pharmaceutical, clinical and research applications. Once substantially purified the fusion protein should be substantially free of contaminants for therapeutic applications. Once purified partially or to substantial purity, the soluble fusion proteins can be used therapeutically, or in performing in vitro or in vivo assays as disclosed herein. Substantial purity can be determined by a variety of standard techniques such as chromatography and gel electrophoresis.

The present fusion protein complexes are suitable for *in vitro* or *in vivo* use with a variety of cells that are cancerous or are infected or that may become infected by one or more diseases.

Human interleukin-15 (hIL-15) is trans-presented to immune effector cells by the human IL-15 receptor α chain (hIL-15Rα) expressed on antigen presenting cells. IL-15Rα binds hIL-15 with high affinity (38 pM) primarily through the extracellular sushi domain (IL-15RaSu). As described herein, the IL-15 and IL-15RaSu domains can be used to generate a soluble complex (e.g., ALT-803) or as a scaffold to construct multi-domain fusion complexes.

IgG domains, particularly the Fc fragment, have been used successfully as dimeric scaffolds for a number of therapeutic molecules including approved biologic drugs. For example, etanercept is a dimer of soluble human p75 tumor necrosis factor-a (TNF-α) receptor (sTNFR) linked to the Fc domain of human IgG1. This dimerization allows etanercept to be up to 1,000 times more potent at inhibiting TNF-α activity than the monomeric sTNFR and provides the fusion with a five-fold longer serum half-life than the monomeric form. As a result, etanercept is effective at neutralization of the pro-inflammatory activity of TNF-α in vivo and improving patient outcome for a number of different autoimmune indications.

In addition to its dimerization activity, the Fc fragment also provides cytotoxic effector functions through the complement activation and interaction with Fcγ receptors displayed on natural killer (NK) cells, neutrophils, monocyte cells, phagocytes and dendritic cells. In the context of anti-cancer therapeutic antibodies and other antibody domain-Fc fusion proteins, these activities likely play an important role in efficacy observed in animal tumor models and in cancer patients. However these cytotoxic effector responses may not be sufficient in a number of therapeutic applications. Thus, there has been considerable interest in improving and expanding on the effector activity of the Fc domain and developing other means of increasing the activity or recruitment of cytolytic immune responses, including NK cells and T cells at the disease site via immunotherapeutic molecules.

In an effort to develop human-derived immunostimulatory therapeutic, human IL-15 (hIL-15) and IL-15 receptor domains were used. hIL-15 is a member of the small four α-helix bundle family of cytokines that associates with the hIL-15 receptor α-chain (hIL-15Rα) with a high binding affinity (Equilibrium dissociation constant (KD) ∼10⁻¹¹ M). The resulting complex is then trans-presented to the human IL-2/15 receptor β/common γ chain (hIL-15RβγC) complexes displayed on the surface of T cells and NK cells. This cytokine/receptor interaction results in expansion and activation of effector T cells and NK cells, which play an important role in eradicating virally infected and malignant cells. Normally, hIL-15 and hIL-15Rα are co-produced in dendritic cells to form complexes intracellularly that are subsequently secreted and displayed as heterodimeric molecules on cell surfaces. Thus, the characteristics of hIL-15 and hIL-15Rα interactions suggest that these inter chain binding domains could serve as a human-derived immunostimulatory complex and as a scaffold to make soluble dimeric molecules capable of target-specific binding.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", second edition (Sambrook, 1989); "Oligonucleotide Synthesis" (Gait, 1984); "Animal Cell Culture" (Freshney, 1987); "Methods in Enzymology" "Handbook of Experimental Immunology" (Weir, 1996); "Gene Transfer Vectors for Mammalian Cells" (Miller and Calos, 1987); "Current Protocols in Molecular Biology" (Ausubel, 1987); "PCR: The Polymerase Chain Reaction", (Mullis, 1994); "Current Protocols in Immunology" (Coligan, 1991). These techniques are applicable to the production of the polynucleotides and polypeptidesdisclosed herein, and, as such, may be considered in making and practicing the invention. Particularly useful techniques for particular embodiments will be discussed in the sections that follow.

Described herein is the enhancement of anti-tumor activities of an anti-CD38 antibody using ALT-803, an IL-15 super-agonist, in in vitro and an experimental animal tumor models. Daratumumab is an anti-CD38 antibody that has recently been approved by FDA for treatment of patients with relapsed or refractory multiple myeloma. Daratumumab has been shown to reduce the tumor burden by targeting CD38 expressing tumor cells. Similarly other anti-CD38 Abs, such as isatuximab (SAR650984), MOR202, Ab79, AB19, and MT-4019, have been shown to be antitumor activity against CD38-epressing tumor cells and/or clinical activity in patients with hematologic tumors. A described in the examples below, the in vitro as well as in vivo ability of ALT-803, a novel IL-15 super-agonist-complex, to enhance the anti-CD38antibody dependent cellular cytotoxicity (ADCC) of daratumumab in combination therapy was examined. Using freshly isolated PBMCs, it was demonstrated that ALT-803 significantly enhanced the ADCC activity of daratumumab against Daudi, human B lymphoma, cells. As described in the examples below, purified human NK cells, which were activated by ALT-803, enhanced daratumumab-mediated ADCC activity against primary human B-lymphoma cells. The in vivo effects of ALT-803 on daratumumab-mediated antitumor activity against Daudi cells were also examined in the SCID xenograft model. It was observed that both ALT-803 and daratumumab alone were capable of reducing Daudi tumor burden in tumor-bearing mice. However, the combination of ALT-803 with daratumumab increased antitumor activity which significantly reduced Daudi cells in the bone marrow. These observations indicate that the addition of ALT-803 to daratumumab treatment enhances the antitumor efficacy of daratumumab compared to daratumumab alone. In addition, as described herein, ALT-803 also augments the Antibody Dependent Cytotoxicity of Phagocytosis of daratumumab against tumor cells. In a current clinical study, the correlative studies also show that ALT-803 augments the ADCC activity of daratumumab against Daudi cells. This also demonstrates that ALT-803 possesses synergistic antitumor activities with tumor-specific antibodies in vivo.

The following examples are put forth so as to provide those of ordinary skill in the art with a complete disclosure and description of how to make and use the therapeutic methods of the invention, and are not intended to limit the scope of what the inventors regard as their invention.

### Example 1: Induction of cell-mediated cytotoxicity by ALT-803 in combination with anti-CD38 antibodies

In this example, "ALT-803" is meant a complex comprising IL-15N72D noncovalently associated with a dimeric IL-15RαSu/Fc fusion protein, wherein said complex exhibits immune stimulating activity. Optionally, the IL-15N72D and/or IL-15RαSu/Fc fusion protein comprises one, two, three, four or more amino acid variations relative to a reference sequence. An exemplary IL-15N72D amino acid sequence is provided below. (See, e.g., U.S.S.N. 13/769,179).

An exemplary IL-15N72D nucleic acid sequence is provided below (with leader peptide) (SEQ ID NO: 1):
(*Leader peptide*)
   atggagacagacacactcctgttatgggtactgctgctctgggttccaggttccaccggt-
*(IL-15N72D)* 
aactgggtgaatgtaataagtgatttgaaaaaaattgaagatcttattcaatctatgcatattgatgctactttatatacggaaagtgatgttc
accccagttgcaaagtaacagcaatgaagtgctttctcttggagttacaagttatttcacttgagtccggagatgcaagtattcatgataca
gtagaaaatctgatcatcctagcaaacgacagtttgtcttctaatgggaatgtaacagaatctggatgcaaagaatgtgaggaactggag
gaaaaaaatattaaagaatttttgcagagttttgtacatattgtccaaatgttcatcaacacttct
*(Stop codon)*
   taa

An exemplary IL-15N72D amino acid sequence is provided below (with leader peptide) (SEQ ID NO: 2):
*(Leader peptide)*
   METDTLLLWVLLLWVPGSTG-
*(IL-15N72D)*

NWVNVISDLKKIEDLIQSMHIDATLYTESDVHPSCKVTAMKCFLLELQVISLESGDAS
IHDTVENLIILANDSLSSNGNVTESGCKECEELEEKNIKEFLQSFVHIVQMFINTS

In some cases, the leader peptide is cleaved from the mature IL-15N72D polypeptide (SEQ ID NO: 3):
*(IL-15N72D)*

NWVNVISDLKKIEDLIQSMHIDATLYTESDVHPSCKVTAMKCFLLELQVISLESGDAS
IHDTVENLIILANDSLSSNGNVTESGCKECEELEEKNIKEFLQSFVHIVQMFINTS

An exemplary IL-15RαSu/Fc nucleic acid sequence (with leader peptide) is provided below (SEQ ID NO: 4):
*(Leader peptide)* atggacagacttacttcttcattcctgctcctgattgtccctgcgtacgtcttgtcc-
*(IL-15RαSu)* 
atcacgtgccctccccccatgtccgtggaacacgcagacatctgggtcaagagctacagcttgtactccagggagcggtacatttgtaa
ctctggtttcaagcgtaaagccggcacgtccagcctgacggagtgcgtgttgaacaaggccacgaatgtcgcccactggacaacccc
cagtctcaaatgtattaga-
*(IgG1 CH2-CH3 (Fc domain))* 
gagcccaaatcttgtgacaaaactcacacatgcccaccgtgcccagcacctgaactcctggggggaccgtcagtcttcctcttcccccc
caaaacccaaggacaccctcatgatctcccggacccctgaggtcacatgcgtggtggtggacgtgagccacgaagaccctgaggtc
aagttcaactggtacgtggacggcgtggaggtgcataatgccaagacaaagccgcgggaggagcagtacaacagcacgtaccgtg
tggtcagcgtcctcaccgtcctgcaccaggactggctgaatggcaaggagtacaagtgcaaggtctccaacaaagccctcccagccc
ccatcgagaaaaccatctccaaagccaaagggcagccccgagaaccacaggtgtacaccctgcccccatcccgggatgagctgac
caagaaccaggtcagcctgacctgcctggtcaaaggcttctatcccagcgacatcgccgtggagtgggagagcaatgggcagccgg
agaacaactacaagaccacgcctcccgtgctggactccgacggctccttcttcctctacagcaagctcaccgtggacaagagcaggt
ggcagcaggggaacgtcttctcatgctccgtgatgcatgaggctctgcacaaccactacacgcagaagagcctctccctgtctccggg
taaa-
*(Stop codon)*
   taa

An exemplary IL-15RαSu/Fc amino acid sequence (with leader peptide) is provided below (SEQ ID NO: 5):
*(Leader peptide)*
   MDRLTSSFLLLIVPAYVLS-
*(IL-15RαSu)* 
ITCPPPMSVEHADIWVKSYSLYSRERYICNSGFKRKAGTSSLTECVLNKATNVAHWT
TPSLKCIR-
*(IgG1 CH2-CH3 (Fc domain))*

EPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSL
SLSPGK

In some cases, the mature IL-15RαSu/Fc protein lacks the leader sequence (SEQ ID NO: 6):
*(IL-15RαSu)* 
ITCPPPMSVEHADIWVKSYSLYSRERYICNSGFKRKAGTSSLTECVLNKATNVAHWT
TPSLKCIR-
*(IgG1 CH2-CH3 (Fc domain))*

EPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSL
SLSPGK

To assess whether ALT-803 in combination with anti-CD38 Ab can affected cell-mediated cytotoxicity against cancer cells, isolated human PBMCs from blood buffy coats were used as effector cells. Daudi human B-cell lymphoma cells were used as target cells and were labeled with CellTrace^{™} Violet at 5 µM in RPMI-10 (RPMI-1640, 2-mercaptoethanol, penicillin-streptomycin-glutamine, non-essential amino acids, sodium pyruvate, and 10% fetal bovine serum) at 37°C for 20 minutes as described by the manufacturer. The effector cells were mixed with the violet-labeled target tumor cells at a ratio of 5:1 and incubated at 37°C with 5% CO₂ in RPMI-10 with and without ALT-803 or with combination with anti-CD38 Ab (daratumumab) for two days. The mixture was then harvested by centrifugation and resuspended in RPMI-10 containing 2 mg/ml propidium iodide. Cytotoxicity of the effector cells against the target cells was evaluated by flow cytometry by determining the percentage of dead violet-labeled target cells after propidium iodide staining. As shown in Figure 1, fresh human PBMCs had weak cytotoxicity against Daudi cells in the absence of ALT-803. In contrast, PBMCs had strong cytotoxicity against Daudi tumor target cells in the presence of ALT-803 at 10 nM. Daudi tumor cells express CD38 molecules, which can be recognized by anti-CD38 Ab (daratumumab). Human PBMCs are capable of lysing Daudi cells by antibody-dependent cellular cytotoxicity (ADCC) mediated by anti-CD38 Ab alone (Figure 1). Importantly, addition of ALT-803 was capable of significantly augmenting anti-CD38 Ab mediated ADCC activity of human PBMCs against Daudi tumor cells.

ALT-803 concentration dependent induction of human PBMC cytotoxicity against Daudi cells was investigated. Fresh human PBMCs were mixed with CellTrace Violet labeled Daudi cells in RPMI-10 medium with various concentrations (from 1 nM to 25 nM) of ALT-803 and a fixed concentration of anti-CD38 Ab (10 nM) followed by incubation for 2 days. The cytotoxicity of the human PBMCs against the target cells was evaluated by flow cytometry as described above. Consistent with the results in Figure 1, ALT-803 was capable of augmenting anti-CD38 Ab-mediated cytotoxicity of human PBMCs against Daudi cells at as low as 1 nM (Figure 2A). Dose responses studies were also conducted in this ADCC assay with varying concentrations of anti-CD38 Ab (from 0.1 nM to 50 nM) and fixed 10 mM ALT-803 (Figure 2B), Increased Daudi cell killing was seen with as little as 0.1 nM anti-CD38 Ab and at every Ab concentration, tumor cell killing was further enhanced by addition of ALT-803.

To identify the immune effector cells responsible for the observed target tumor cell killing, NK cells were isolated from human PBMCs by MACS and used as the effector cells in the ADCC assay described above. Similarly, to results with total human PBMC, NK cells were capable of killing Daudi tumor cells via anti-CD38 Ab-mediated ADCC activity that was enhanced by addition of ALT-803 (Figure 2C). Compare to total PBMCs, greater cytotoxicity against tumor targets was observed with NK cell incubated with ALT-803 plus anti-CD38 Ab.

### Example 2: Antitumor activity of ALT-803 plus anti-CD38 Ab treatment in mice bearing hematologic tumors

The ability of ALT-803 to augment the antitumor efficacy of anti-CD38 Ab was further evaluated in SCID mice bearing Daudi tumors. In addition, effect of the combination of ALT-803 plus anti-CD38 Ab was assessed in tumor-bearing mice following pretreatment with steroids. Steroids are commonly used in conjunction with anti-CD38 Ab therapy as antiinflammatory agents to reduce infusion reactions. However, there is also concern that the immunosuppressive effects of these steroids may also limit the immune-mediated efficacy of the anti-CD38 Ab therapy. For this study, Fox Chase SCID female mice (Harlan, C.B-17/IcrHsd-Prkdc-scid: 6 weeks-old) were inoculated intravenously (i.v.) with Daudi cells (10 × 10⁶ per mouse) (6 mice/group). These mice have functional NK cells which likely mediate ADCC responses against tumors. In some groups, tumor-bearing mice were also treated on day 15 (post tumor inoculation) with steroid (hydrocortisone, 0.2 mg/mouse, intra-peritoneal) one hour prior to i.v. treatment with PBS or anti-CD38 Ab (10 mg/kg). On day 17, after two days of either PBS or anti-CD38 Ab treatment, depend upon the treatment group, mice were further treated with ALT-803 (0.2 mg/kg, subcutaneously). Seven days after anti-CD38 Ab treatment the mice were sacrificed and the levels of the Daudi cells in bone marrow were determined. The percentage of Daudi cells of femur bone marrow cells was assessed following staining with PE-conjugated anti-human HLA-DR antibody (BioLegend) and flow cytometry analysis. As shown in Figure 3, ALT-803 and anti-CD38 Ab monotherapies are capable of reducing the percentage of Daudi cells in the bone marrow of tumor-bearing mice. However, the combination of ALT-803 plus anti-CD38 Ab provided the greatest antitumor activity reducing bone marrow Daudi cells from 62% in control mice to 10% in ALT-803 + anti-CD38 Ab treated mice. This enhance efficacy was also observed in tumor-bearing mice treated with steroids prior to ALT-803 plus anti-CD38 Ab therapy. In fact, similar antitumor activity was seen in ALT-803 plus anti-CD38 Ab treated mice with or without pre-treatment with steroids.

### Example 3. ALT-803 in combination with anti-CD38 Ab prolongs survival of mice bearing hematologic tumors

The ability of ALT-803 plus anti-CD38 Ab to prolong survival of mice bearing Daudi cell tumors was also evaluated. For this study, Fox Chase SCID female mice were inoculated intravenously (i.v.) with Daudi cells (10 × 10⁶ per mouse). Fifteen days later, the tumor-bearing mice were treated with either PBS or and anti-CD38 Ab (10 mg/kg, i.v.). All mice were pre-treated with steroid (hydrocortisone, 0.2 mg/mouse, intra-peritoneal) to reduce infusion reaction, except the PBS treated mice. On day 17 ALT-803 and ALT-803 combination with anti-CD38 Ab groups mice were treated with ALT-803 (0.2 mg/kg, subcutaneously). On day 22, the ALT-803, anti-CD38 Ab and ALT-803 with combination with anti-CD38 Ab groups were treated similar as on day 15 and day 17. Survival (including morbidity based on hind leg paralysis) of the mice was monitored. As shown in Figure 4, tumor bearing mice treated with PBS, ALT-803 and anti-CD38 Ab monotherapy groups showed median survival of 31- 41 days whereas all tumor-bearing mice treated with anti-CD38 Ab survived greater than 45 days, indicating combined therapy significantly prolongs survival of mice bearing B cell lymphomas. Together, these results confirm the synergistic antitumor effects of ALT-803 in combination with anti-CD38 Ab in vivo. These results were observed in the presence of steroid pretreatment.

### Example 4. ALT-803-stimulated NK cells enhance anti-CD38 mediated macrophage activity against tumor cells

Therapeutic antibodies may mediate antitumor activity in part through antibody-dependent cellular phagocytic (ADCP) activity of macrophages against tumor cells. Macrophage activity in turn can be stimulated by cytokines and other agents release by other immune effector cells. To access the ability of ALT-803 to affect antitumor activity of macrophage, we set up a flow cytometric based phagocytosis assay using human macrophage cell line THP-1 as primary effector cells, human NK-92 cell line (NK cell line) as secondary effector cells and Daudi cells as target tumor cells. In this study, a Transwell^{™} system was used to separate NK-92 cells (aNK cells) from THP-1 and Daudi cells. NK-92 cells (1 × 10⁵) were cultured in top wells of Transwell^{™} insert (Corning) whereas THP-1 cells (1 × 10⁵) (labeled with PKH67 as per manufactured protocol-Sigma) and Daudi cells (1 × 10⁵) (labeled with CellTrace Violet as per manufactured protocol-Molecular Probes) in bottom wells of 24 wells plate. Cells were either treated with media alone or media containing 10 nM ALT-803, 10 nM anti-CD38 Ab, or 10 nM ALT-803 plus 10 nM anti-CD38 Ab in 1 ml total volume in each well. After 24 hours, THP-1 and Daudi cells were harvested by centrifugation and resuspended in flow-cytometer buffer for assessed the phagocytosis by determining the percentage of cells labeled with PKH67 and CellTrace Violet (representative flow cytometry plots shown in Fig. 5). Such double positive cells represent the PKH67-labeled THP-1 macrophage that have phagocytosed CellTrace Violet-labeled Daudi cells. The combination of ALT-803 plus anti-CD38 Ab increased macrophage-mediated phagocytosis of tumor cells, as shown by an increase in the percentage of PKH67⁺ CellTrace Violet⁺ cells (Fig. 5), when compare to when compared to cells incubated with ALT-803 or anti-CD38 Ab alone. This enhanced activity was dependent on the presence of NK cells and ALT-803 suggesting that ALT-803-dependent activation of NK cells results in stimulation of Ab-directed macrophage phagocytosis of diseased cells, e.g. tumor cells.

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

The patent and scientific literature referred to herein establishes the knowledge that is available to those with skill in the art.

While this invention has been particularly shown and described with references to preferred embodiments thereof, it will be understood by those skilled in the art that various changes in form and details may be made therein without departing from the scope of the invention encompassed by the appended claims.

## Claims

1. A combination of (i) an anti-CD38 antibody or antibody-like molecule and (ii) an IL-15:IL-15Rα complex for use in a method for treating a neoplasia in a subject, the method comprising:
administering to said subject 1) an effective amount of the anti-CD38 antibody or antibody-like molecule and 2) an effective amount of the IL-15:IL-15Rα complex,
thereby treating the neoplasia.

2. The combination for the use of claim 1 wherein the IL-15:IL-15Rα complex comprises a complex (ALT-803) between a dimeric IL-15RαSu/Fc molecule and two IL-15N72D molecules.

3. The combination for the use of any one of claims 1-2 further comprising administering to the subject a steroid before, during or after administration of the antibody or antibody-like molecule or the IL-15:IL-15Rα complex, preferably wherein the steroid is selected from the group comprising corticosteroids, antipyretics and antihistamines.

4. The combination for the use of any one of claims 2-3 wherein the IL-15N72D molecule comprises SEQ ID NO: 3 and/or wherein the IL-15RαSu/Fc comprises SEQ ID NO: 6.

5. The combination for the use of any one of claims 1-4 wherein the neoplasia is selected from the group consisting of a hematological cancer, B-cell neoplasm, multiple myeloma (MM), chronic lymphocytic leukemia (CLL), B and T acute lymphocytic leukemia, acute myeloid leukemia, chronic myeloid leukemia, hairy cell leukemia, Waldenstrom's macroglobulinemia, primary systemic amyloidosis, mantle cell lymphoma, NK cell leukemia, NK or T-cell lymphoma, plasma cell leukemia, B-cell lymphoma, non-Hodgkin's lymphoma, Burkitt's lymphoma, Hodgkin's lymphoma, and solid tumor.

6. The combination for the use of any one of claims 1-5 wherein the effective amount of the IL-15:IL-15Rα complex is administered daily, once or twice per week or once or twice per month.

7. The combination for the use of any one of claims 1-6 wherein the effective amount of the IL-15:IL-15Rα complex is between 0.1 µg/kg and 100 mg/kg.

8. The combination for the use of any one of claims 1-7 wherein the anti-CD38 antibody is daratumumab, isatuximab (SAR650984), or MOR202.

9. The combination for the use of any one of claims 1-8 wherein antibody-dependent cell-mediated cytotoxicity (ADCC) or antibody dependent cellular phagocytosis (ADCP) mediated by said antibody against tumor cells in said subject is augmented by at least 5% following said administration.

10. The combination for the use of any one of claims 1-9 wherein the antibody or antibody-like molecule and the IL-15:IL-15Rα complex
- increase levels of blood NK cell, T cell, neutrophil, macrophage, dendritic cell or monocyte counts or activity; and/or
- stimulate CD4⁺ or CD8⁺ T cells to kill tumor cells; and/or
- stimulate NK cells to kill tumor cells; and/or
- stimulate neutrophils or monocytic cells to kill tumor cells.

11. The combination for the use of any one of claims 1-10 wherein the administration of the antibody or antibody-like molecule and the IL-15:IL-15Rα complex
- results in a decrease in the number of tumor cells; and/or
- results in a decrease in disease progression of the neoplasia.

12. The combination for the use of any one of claims 1-11 wherein the antibody or antibody-like molecule and the IL-15:IL-15Rα complex are administered simultaneously or sequentially.

13. A kit or a pharmaceutical composition comprising 1) an anti-CD38 antibody or antibody-like molecule and 2) an IL-15:IL-15Rα complex.

14. The kit or pharmaceutical composition of claim 13 wherein the IL-15:IL-15Ra complex comprises a complex (ALT-803) between a dimeric IL-15RαSu/Fc molecule and two IL-15N72D molecules.

## Patentansprüche

1. Kombination aus (i) einem Anti-CD38-Antikörper oder antikörperähnlichen Molekül und (ii) einem IL-15:IL-15Rα-Komplex zur Verwendung in einem Verfahren zum Behandeln einer Neoplasie bei einem Subjekt, wobei das Verfahren Folgendes umfasst:
Verabreichen, an das Subjekt, 1) einer wirksamen Menge des Anti-CD38-Antikörpers oder des antikörperähnlichen Moleküls und 2) einer wirksamen Menge des IL-15:IL-15Rα-Komplexes, wodurch die Neoplasie behandelt wird.

2. Kombination zur Verwendung nach Anspruch 1, wobei der IL-15:IL-15Rα-Komplex einen Komplex (ALT-803) zwischen einem dimeren IL-15RαSu/Fc-Molekül und zwei IL-15N72D-Molekülen umfasst.

3. Kombination zur Verwendung nach einem der Ansprüche 1-2, ferner umfassend das Verabreichen eines Steroids an das Subjekt vor, während oder nach dem Verabreichen des Antikörpers oder des antikörperähnlichen Moleküls oder des IL-15:IL-15Rα-Komplexes, vorzugsweise wobei das Steroid ausgewählt ist aus der Gruppe bestehend aus Corticosteroiden, Antipyretika und Antihistaminika.

4. Kombination zur Verwendung nach einem der Ansprüche 2-3, wobei das IL-15N72D-Molekül SEQ ID NO: 3 umfasst und/oder wobei das IL-15RαSu/Fc SEQ ID NO: 6 umfasst.

5. Kombination zur Verwendung nach einem der Ansprüche 1-4, wobei die Neoplasie ausgewählt ist aus der Gruppe bestehend aus einem hämatologischen Krebs, B-Zell-Neoplasma, multiplem Myelom (MM), chronischer lymphatischer Leukämie (CLL), akuter lymphatischer B- und T-Zell-Leukämie, akuter myeloischer Leukämie, chronischer myeloischer Leukämie, Haarzell-Leukämie, Waldenström-Makroglobulinämie, primärer systemischer Amyloidose, Mantelzell-Lymphom, NK-Zell-Leukämie, NK- oder T-Zell-Lymphom, Plasmazell-Leukämie, B-Zell-Lymphom, Non-Hodgkin-Lymphom, Burkitt-Lymphom, Hodgkin-Lymphom und solidem Tumor.

6. Kombination zur Verwendung nach einem der Ansprüche 1 bis 5, wobei die wirksame Menge des IL-15:IL-15Rα-Komplexes täglich, ein- oder zweimal pro Woche oder ein- oder zweimal pro Monat verabreicht wird.

7. Kombination zur Verwendung nach einem der Ansprüche 1-6, wobei die wirksame Menge des IL-15:IL-15Rα-Komplexes zwischen 0,1 µg/kg und 100 mg/kg liegt.

8. Kombination zur Verwendung nach einem der Ansprüche 1-7, wobei der Anti-CD38-Antikörper Daratumumab, Isatuximab (SAR650984) oder MOR202 ist.

9. Kombination zur Verwendung nach einem der Ansprüche 1-8, wobei die antikörperabhängige zellvermittelte Zytotoxizität (ADCC) oder die antikörperabhängige zelluläre Phagozytose (ADCP), die durch den Antikörper gegen Tumorzellen in dem Subjekt vermittelt wird, nach dem Verabreichen um mindestens 5 % erhöht ist.

10. Kombination zur Verwendung nach einem der Ansprüche 1-9, wobei der Antikörper oder das antikörperähnliche Molekül und der IL-15:IL-15Rα-Komplex
- die Anzahl oder Aktivität von NK-Zellen, T-Zellen, Neutrophilen, Makrophagen, dendritischen Zellen oder Monozyten im Blut erhöhen; und/oder
- CD4⁺- oder CD8⁺-T-Zellen stimulieren, um Tumorzellen abzutöten; und/oder
- NK-Zellen stimulieren, um Tumorzellen abzutöten; und/oder
- neutrophile oder monozytäre Zellen stimulieren, um Tumorzellen abzutöten.

11. Kombination zur Verwendung nach einem der Ansprüche 1 bis 10, wobei das Verabreichen des Antikörpers oder des antikörperähnlichen Moleküls und des IL-15:IL-15Rα-Komplexes
- zu einer Verringerung der Anzahl von Tumorzellen führt; und/oder
- zu einer Abschwächung des Krankheitsverlaufs der Neoplasie führt.

12. Kombination zur Verwendung nach einem der Ansprüche 1 bis 11, wobei der Antikörper oder das antikörperähnliche Molekül und der IL-15:IL-15Rα-Komplex gleichzeitig oder nacheinander verabreicht werden.

13. Kit oder pharmazeutische Zusammensetzung, umfassend 1) einen Anti-CD38-Antikörper oder ein antikörperähnliches Molekül und 2) einen IL-15:IL-15Rα-Komplex.

14. Kit oder pharmazeutische Zusammensetzung nach Anspruch 13, wobei der IL-15:IL-15Rα-Komplex einen Komplex (ALT-803) zwischen einem dimeren IL-15RαSu/Fc-Molekül und zwei IL-15N72D-Molekülen umfasst.

## Revendications

1. Combinaison de (i) une molécule d'anticorps ou de type anticorps anti-CD38 et (ii) un complexe d'IL-15 : IL-15Rα pour une utilisation dans un procédé pour le traitement d'une néoplasie chez un sujet, le procédé comprenant :
l'administration audit sujet 1) d'une quantité efficace de la molécule d'anticorps ou de type anticorps anti-CD38 et 2) une quantité efficace du complexe d'IL-15 : IL-15Rα, traitant ainsi la néoplasie.

2. Combinaison pour l'utilisation selon la revendication 1, le complexe d'IL-15 : IL-15Rα comprenant un complexe (ALT-803) entre une molécule d'IL-15RαSu/Fc dimérique et deux molécules d'IL-15N72D.

3. Combinaison pour l'utilisation selon l'une quelconque des revendications 1 et 2 comprenant en outre l'administration au sujet d'un stéroïde avant, pendant ou après l'administration de la molécule d'anticorps ou de type anticorps ou du complexe d'IL-15 : IL-15Rα, préférablement le stéroïde étant choisi dans le groupe comprenant des corticostéroïdes, des antipyrétiques et des antihistaminiques.

4. Combinaison pour l'utilisation selon l'une quelconque des revendications 2 et 3, la molécule d'IL-15N72D comprenant la SEQ ID NO: 3 et/ou l'IL-15RαSu/Fc comprenant la SEQ ID NO: 6.

5. Combinaison pour l'utilisation selon l'une quelconque des revendications 1 à 4, la néoplasie étant choisie dans le groupe constitué par un cancer hématologique, un néoplasme à cellules B, un myélome multiple (MM), une leucémie lymphocytaire chronique (LLC), une leucémie lymphocytaire aiguë B et T, une leucémie myéloïde aiguë, une leucémie myéloïde chronique, une leucémie à tricholeucocytes, une macroglobulinémie de Waldenström, une amyloïdose systémique primaire, un lymphome à cellules du manteau, une leucémie à cellules NK, un lymphome à cellules NK ou T, une leucémie plasmocytaire, un lymphome à cellules B, un lymphome non hodgkinien, un lymphome de Burkitt, un lymphome hodgkinien et une tumeur solide.

6. Combinaison pour l'utilisation selon l'une quelconque des revendications 1 à 5, la quantité efficace du complexe d'IL-15 : IL-15Rα étant administrée quotidiennement, une ou deux fois par semaine ou une ou deux fois par mois.

7. Combinaison pour l'utilisation selon l'une quelconque des revendications 1 à 6, la quantité efficace du complexe d'IL-15 : IL-15Rα étant entre 0,1 µg/kg et 100 mg/kg.

8. Combinaison pour l'utilisation selon l'une quelconque des revendications 1 à 7, l'anticorps anti-CD38 étant le daratumumab, l'isatuximab (SAR650984) ou MOR202.

9. Combinaison pour l'utilisation selon l'une quelconque des revendications 1 à 8, une cytotoxicité à médiation cellulaire dépendante des anticorps (ADCC) ou une phagocytose cellulaire dépendante des anticorps (ADCP) médiée par ledit anticorps contre des cellules tumorales chez ledit sujet étant augmentée d'au moins 5 % à la suite de ladite administration.

10. Combinaison pour l'utilisation selon l'une quelconque des revendications 1 à 9, la molécule d'anticorps ou de type anticorps et le complexe d'IL-15 : IL-15Rα
- augmentant des niveaux de quantité ou d'activité de cellules NK, de cellules T, de neutrophiles, de macrophages, de cellules dendritiques ou de monocytes dans le sang ; et/ou
- stimulant des cellules CD4⁺ ou CD8⁺T pour tuer des cellules tumorales ; et/ou
- stimulant des cellules NK pour tuer des cellules tumorales ; et/ou
- stimulant des neutrophiles ou des cellules monocytaires pour tuer des cellules tumorales.

11. Combinaison pour l'utilisation selon l'une quelconque des revendications 1 à 10, l'administration de la molécule d'anticorps ou de type anticorps et du complexe d'IL-15 : IL-15Rα
- entraînant une diminution du nombre de cellules tumorales ; et ou
- entraînant une diminution de la progression de la maladie de la néoplasie.

12. Combinaison pour l'utilisation selon l'une quelconque des revendications 1 à 11, la molécule d'anticorps ou de type anticorps et le complexe d'IL-15 : IL-15Rα étant administrés simultanément ou séparément.

13. Kit ou composition pharmaceutique comprenant 1) une molécule d'anticorps ou de type anticorps anti-CD38 et 2) un complexe d'IL-15 : IL-15Rα.

14. Kit ou composition pharmaceutique selon la revendication 13, le complexe d'IL-15 : IL-15Rα comprenant un complexe (ALT-803) entre une molécule d'IL-15RαSu/Fc dimérique et deux molécules d'IL-15N72D.
